# EUROPEAN PATENT SPECIFICATION

(11) **EP 0 585 398 B1**
(45) Date of publication and mention of the grant of the patent: **15.10.2003**
(21) Application number: 92913881.6
(22) Date of filing: 08.05.1992
(51) Int. Cl.: C12N 15/51, C12Q 1/68, C12N 15/40, C12Q 1/70, G01N 33/576

(54) **HCV GENOMIC SEQUENCES FOR DIAGNOSTICS AND THERAPEUTICS**
GENOMISCHE HCV-SEQUENZEN FÜR DIAGNOSTIK UND THERAPIE
SEQUENCES GENOMIQUES DU VIRUS DE L'HEPATITE C UTILISEES EN DIAGNOSTIC ET EN THERAPEUTIQUE

(30) Priority: 08.05.1991 US 697326
(43) Date of publication of application: 09.03.1994
(60) Divisional application: 03013389.6
(73) Proprietor: CHIRON CORPORATION, Emeryville, California 94608 (US)
(72) Inventor: CHA, Tai-An, San Ramon, CA 94583 (US); BEALL, Eileen, Walnut Creek, CA 94596 (US); IRVINE, Bruce, Concord, CA 94519 (US); KOLBERG, Janice, Hercules, CA 94547 (US); URDEA, Michael, S., Alamo, CA 94501 (US)
(74) Representative: UEXKÜLL & STOLBERG
(86) International application number: US9204036
(87) International publication number: WO92019743

(56) References cited:
- EP-A- 0 388 232
- EP-A- 0 463 848
- EP-A- 0 464 287
- WO-A-91/14779
- WO-A-91/15516
- GB-A- 2 239 245
- BIOCHEM. BIOPHYS. RES. COMMUN. vol. 170, no. 3, 1990, pages 1021 - 1025 N. ENOMOTO ET AL. 'There are two major types of hepatitis c in Japan'
- PROC. NAT'L. ACAD. SCI. USA vol. 88, 1991, pages 3392 - 3396 N. OGATA ET AL. 'Nucleotide sequence and mutation rate of the H strain of hepatitis C virus'
- JAPAN. J. EXP. MED. vol. 60, no. 3, 1990, pages 167 - 177 H. OKAMOTO ET AL. 'The 5' terminal sequence of the hepatitis C virus genome'
- PROC. NAT'L. ACAD. SCI. USA vol. 88, 1991, pages 2451 - 2455 Q. L. CHOO ET AL. 'Genetic organisation and diversity of the hepatitis C virus'
- VIROLOGY vol. 180, 1991, pages 842 - 848 A.WEINER ET AL. 'Variable and hypervariable domains are found in the regions of HCV corresponding to the flavivirus envelope and NS1 proteins'

## Description

### Technical Field

The invention relates to compositions and methods for the detection and treatment of hepatitis C virus, (HCV) infection, formerly referred to as blood-borne non-A, non-B hepatitis virus (NANBV) infection. More specifically, embodiments of the present invention feature compositions and methods for the detection of HCV, and for the development of vaccines for the prophylactic treatment of infections of HCV, and development of antibody products for conveying passive immunity toHCV.

### Background of the Invention

The prototype isolate of HCV was characterized in U.S. Patent Application Serial No. 122,714 (See also EPO Publication No. 318,216). As used herein, the term "HCV" includes new isolates of the same viral species. The term "HCV-1" referred to in U.S. Patent Application Serial No. 122,714. HCV is a transmissible disease distinguishable from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). HCV was first identified in individuals who had received blood transfusions.

The demand for sensitive, specific methods for screening and identifying carriers of HCV and HCV contaminated blood or blood products is significant. Post-transfusion hepatitis (PTH) occurs in approximately 10% of transfused patients, and HCV accounts for up to 90% of these cases. The disease frequently progresses to chronic liver damage (25-55%).

Patient care as well as the prevention of transmission of HCV by blood and blood products or by close personal contact require reliable screening, diagnostic and prognostic tools to detect nucleic acids, antigens and antibodies related to HCV.

Information in this application suggests the HCV has several genotypes. That is, the genetic information of the HCV virus may not be totally identical for all HCV, but encompasses groups with differing genetic information.

Genetic information is stored in thread-like molecules of DNA and RNA. DNA consists of covalently linked chains of deoxyribonucleotides and RNA consists of covalently linked chains of ribonucleotides. Each nucleotide is characterized by one of four bases: adenine (A), guanine (G), thymine (T), and cytosine (C). The bases are complementary in the sense that, due to the orientation of functional groups, certain base pairs attract and bond to each other through hydrogen bonding and π-stacking interactions. Adenine in one strand of DNA pairs with thymine in an opposing complementary strand. Guanine in one strand of DNA pairs with cytosine in an opposing complementary strand. In RNA, the thymine base is replaced by uracil (U) which pairs with adenine in an opposing complementary strand. The genetic code of living organism is carried in the sequence of base pairs. Living cells interpret, transcribe and translate the information of nucleic acid to make proteins and peptides.

The HCV genome is comprised of a single positive strand of RNA. The HCV genome possesses a continuous, translational open reading frame (ORF) that encodes a polyprotein of about 3,000 amino acids. In the ORF, the structural protein(s) appear to be encoded in approximately the first quarter of the N-terminus region, with the majority of the polyprotein responsible for non-structural proteins.

The HCV polyprotein comprises, from the amino terminus to the carboxy terminus, the nucleocapsid protein (C), the envelope protein (E), and the non-structural proteins (NS) 1, 2 (b), 3, 4 (b), and 5.

HCV of differing genotypes may encode for proteins which present an altered response to host immune systems. HCV of differing genotypes may be difficult to detect by immuno diagnostic techniques and nucleic acid probe techniques which are not specifically directed to such genotype.

Definitions for selected terms used in the application are set forth below to facilitate an understanding of the invention. The term "corresponding" means homologous to or complementary to a particular sequence of nucleic acid. As between nucleic acids and peptides, corresponding refers to amino acids of a peptide in an order derived from the sequence of a nucleic acid or its complement.

The term "non-naturally occurring nucleic acid" refers to a portion of genomic nucleic acid, cDNA, semisynthetic nucleic acid, or synthetic origin nucleic acid which, by virtue of its origin or manipulation: (1) is not associated with all of a nucleic acid with which it is associated in nature, (2) is linked to a nucleic acid or other chemical agent other than that to which it is linked in nature, or (3) does not occur in nature.

Similarly the term, "a non-naturally occurring peptide" refers to a portion of a large naturally occurring peptide or protein, or semi-synthetic or synthetic peptide, which by virtue of its origin or manipulation (1) is not associated with all of a peptide with which it is associated in nature, (2) is linked to peptides, functional groups or chemical agents other than that to which it is linked in nature, or (3) does not occur in nature.

The term "primer" refers to a nucleic acid which is capable of initiating the synthesis of a larger nucleic acid when placed under appropriate conditions. The primer will be completely or substantially complementary to a region of the nucleic acid to be copied. Thus, under conditions conducive to hybridization, the primer will anneal to a complementary region of a larger nucleic acid. Upon addition of suitable reactants, the primer is extended by the polymerizing agent to form a copy of the larger nucleic acid.

The term "binding pair" refers to any pair of molecules which exhibit mutual affinity or binding capacity. For the purposes of the present application, the term "ligand" will refer to one molecule of the binding pair, and the term "antiligand" or "receptor" or "target" will refer to the opposite molecule of the binding pair. For example, with respect to nucleic acids, a binding pair may comprise two complementary nucleic acids. One of the nucleic acids may be designated the ligand and the other strand is designated the antiligand receptor or target. The designation of ligand or antiligand is a matter of arbitrary convenience. Other binding pairs comprise, by way of example, antigens and antibodies, drugs and drug receptor sites and enzymes and enzyme substrates, to name a few.

The term "label" refers to a molecular moiety capable of detection including, by way of example, without limitation, radioactive isotopes, enzymes, luminescent agents, precipitating agents, and dyes.

The term '"support" includes conventional supports such as filters and membranes as well as retrievable supports which can be substantially dispersed within a medium and removed or separated from the medium by immobilization, filtering, partitioning, or the like. The term "support means" refers to supports capable of being associated to nucleic acids, peptides or antibodies by binding partners, or covalent or noncovalent linkages.

A number of HCV strains and isolates have been identified. When compared with the sequence of the original isolate derived from the USA ("HCV-1"; see Q.-L. Choo et al. (1989) Science 244:359-362, Q.-L. Choo et al. (1990) Brit. Med. Bull. 46:423-441, Q.-L. Choo et al., Proc. Natl. Acad. Sci. 88:2451-2455 (1991), and E.P.O. Patent Publication No. 318,216, cited supra), it was found that a Japanese isolate ("HCV J1") differed significantly in both nucleotide and polypeptide sequence within the NS3 and NS₄ regions. This conclusion was later extended to the NS5 and envelope (El/S and E2/NS1) regions (see K. Takeuchi et al., J. Gen. Virol. (1990) 71:3027-3033, Y. Kubo. Nucl. Acids. Res. (1989) 17:10367-10372, and K. Takeuchi et al., Gene (1990) 91:287-291). The former group of isolates, originally identified in the United States, is termed "Genotype I"throughout the present disclosure, while the latter group of isolates, initially identified in Japan, is termed "Genotype II" herein.

### Brief Description of the Invention

The present invention features compositions of matter comprising nucleic acids corresponding to the HCV viral genome which define different genotypes. The present invention also features methods of using the compositions corresponding to sequences of the HCV viral genome which define different genotypes described herein.

### A. Nucleic acid compositions

The nucleic acid of the present invention, corresponding to the HCV viral genome which define different genotypes, having utility as probes in nucleic acid hybridization assays, as primers for reactions involving the synthesis of nucleic acid, as binding partners for separating HCV viral nucleic acid from other constituents which may be present, and as anti-sense nucleic acid for preventing the transcription or translation of viral nucleic acid.

One embodiment of the present invention features a composition comprising a non-naturally occurring nucleic acid comprising a non-HCV-1 nucleotide consisting of from eight to 252 contiguous nucleotides which are fully homologous to or fully complementary to a nucleotide sequence from the 5'UT region of a hepatitis C virus and selected from a sequence within SEQ ID NOs:34 to 49 and wherein said sequence is not HCV-J1 or HSC-J4.

Preferably, with respect to the sequences which correspond to the 5'UT regions, the sequence is selected from a sequence within sequences numbered 34-51. Sequence No. 33 corresponds to HCV-1. Sequence No. 33-51 are set forth in the Sequence Listing of this application.

The compositions of the present invention form hybridization products with nucleic acid corresponding to different genotypes of HCV.

HCV has at least five genotypes, which will be referred to in this application by the designations GI-GV. The first genotype, GI, is exemplified by sequences numbered 33-38 The second genotype, GII, is exemplified by the sequences numbered 39-45. The third genotype, GIII, is exemplified by sequences numbered 46-47 The fourth genotype, GIV, is exemplified by sequences numbered

48-49. The fifth denotype, GV, is exemplified by sequences numbered 50 and 51.

One embodiment of the present invention features compositions comprising a nucleic acid having a sequence corresponding to one or more sequences which exemplify a genotype of HCV.

### B. Method of forming a Hybridization Product

Embodiments of the present invention also feature a method of forming a hybridization product with nucleic acid having a sequence corresponding to HCV nucleic acid. One method comprises the steps of placing a non-naturally occurring nucleic acid having a Don-HCV-1 sequence corresponding to HCV nucleic acid under conditions in which hybridization may occur. The non-naturally occurring nucleic acid is capable of forming a hybridization product with HCV nucleic acid, under hybridization conditions. The method further comprises the step of imposing hybridization conditions to form a hybridization product in the presence of nucleic acid corresponding to a region of the HCV genome.

The formation of a hybridization product has utility for detecting the presence of one or more genotypes of HCV. Preferably, the non-naturally occurring nucleic acid forms a hybridization product with nucleic acid of HCV in one or more regions comprising the NS5 region, envelope 1 region, 5'UT region and the core region. To detect the hybridization product, it is useful to associate the non-naturally occurring nucleic acid with a label. The formation of the hybridization product is detected by separating the hybridization product from labeled non-naturally occurring nucleic acid, which has not formed a hybridization product.

The formation of a hybridization product has utility as a means of separating one or more genotypes of HCV nucleic acid from other constituents potentially present. For such applications, it is useful to associate the non-naturally occurring nucleic acid with a support for separating the resultant hybridization product from the the other constituents.

Nucleic acid "sandwich assays" employ one nucleic acid associated with a label and a second nucleic acid associated with a support. An embodiment of the present invention features a sandwich assay comprising two nucleic acids, both have sequences which correspond to HCV nucleic acids; however, at least one non-naturally occurring nucleic acid has a sequence corresponding to non-HCV-1 HCV nucleic acid. At least one nucleic acid is capable of associating with a label, and the other is capable of associating with a support. The support associated non-naturally occurring nucleic acid is used to separate the hybridization products which include an HCV nucleic acid and the non-naturally occurring nucleic acid having a non-HCV-1 sequence.

one embodiment of the present invention features a method of detecting one or more genotypes of HCV. The method comprises the steps of placing a non-naturally occurring nucleic acid under conditions which hybridization may occur. The non-naturally occurring nucleic acid is capable of forming a hybridization product with nucleic acid from one or more genotypes of HCV. The first genotype, GI, is exemplified by sequences numbered 33-38 The second genotype, GII, is exemplified by the sequences numbered 39-45 The third genotype. GIII, is exemplified by sequences numbered

46-47 The fourth genotype. GIV, is exemplified sequences numbered . 48-49. The fifth genotype, GV, is exemplified by sequences numbered 50 and 51.

The nybridization product of HCV nucleic acid with a non-naturally occurring nucleic acid having non-HCV-1 sequence corresponding to sequences within the HCV genome has utility for priming a reaction for the synthesis of nucleic acid.

The hybridization product of HCV nucleic acid with a non-naturally occurring nucleic acid having a sequence corresponding to a particular genotype of HCV has utility for priming a reaction for the synthesis of nucleic acid of such genotype. In one embodiment, the synthesized nucleic acid is indicative of the presence of one or more genotypes of HCV.

The synthesis of nucleic acid may also facilitate cloning of the nucleic acid into expression vectors which synthesize viral proteins.

Embodiments of the present methods have utility as anti-sense agents for preventing the transcription or translation of viral nucleic acid. The formation of a hybridization product of a non-naturally occurring nucleic acid having sequences which correspond to a particular genotype of HCV genomic sequencing with HCV nucleic acid may block translation or transcription of such genotype. Therapeutic agents can be engineered to include all five genotvpes for inclusivity

The present invention is further described in the following figures which illustrate sequences demonstrating genotypes of HCV. The sequences are designated by numerals 1-145, which numerals and sequences are consistent with the numerals and sequences set forth in the Sequence Listing. Sequences 146 and 147 facilitate the discussion of an assay which numerals and sequences are consistent with the numerals and sequences set forth in the Sequence Listing.

### Brief Description of the Figures and Sequence Listing

Figure 1 depicts schematically the genetic organization of HCV;

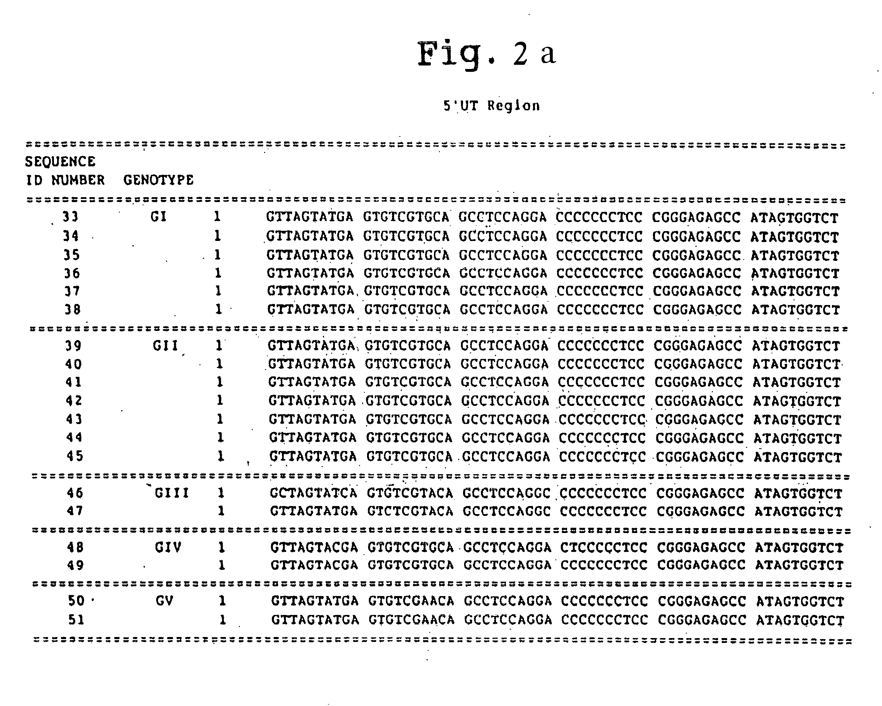

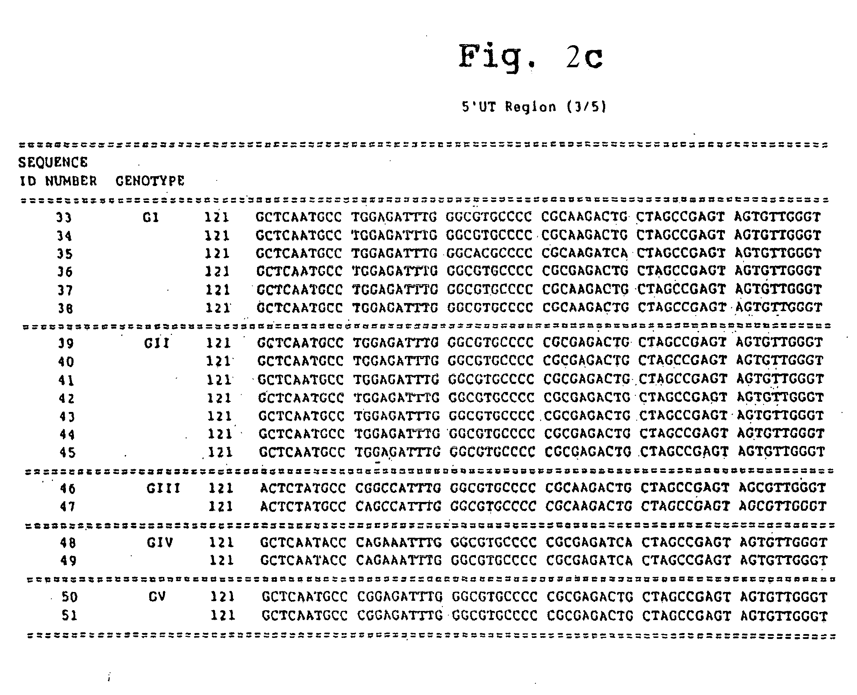

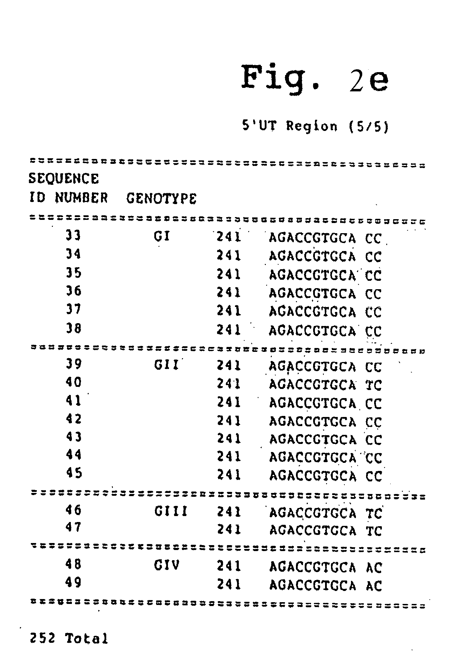

Figure 2 sets forth nucleic acid sequences numbered 33-51 which sequences are derived from the 5 UT region of the HCV viral genome ; and,

The Sequence Listing sets forth the sequences of sequences numbered 1-147.

### Detailed Description of the Invention

The present invention will be described in detail as as nucleic acid having sequences corresponding to the HCV genome and related peptides and binding partners, for diagnostic and therapeutic applications.

The practice of the present invention will employ, unless otherwise indicated, conventional techniques of chemistry, molecular biology, microbiology, recombinant DNA, and immunology, which are within the skill of the art. Such techniques are explained fully in the literature. See e.g., Maniatis, Fitsch & Sambrook, Molecular Cloning; A Laboratory Manual (1982); DNA Cloning, Volumes I and II (D.N Glover ed. 1985); oligonucleotide Synthesis (M.J. Gait ed, 1984); Nucleic Acid Hybridization (B.D. Hames & S.J. Higgins eds. 1984); the series, Methods in Enzymology (Academic Press, Inc.), particularly Vol. 154 and Vol. 155 (Wu and Grossman, eds.).

The cDNA libraries are derived from nucleic acid sequences present in the plasma of an HCV-infected chimpanzee. The construction of one of these libraries, the "c" library (ATCC No. 40394), is described in PCT Pub. No. W090/14436. The sequences of the library relevant to the present invention are set forth herein as sequence numbers 1, 23, 33 and 52.

Nucleic acids isolated or synthesized in accordance with features of the present invention are useful, by way of example without limitation as probes, primers, anti-sense genes and for developing expression systems for the synthesis of peptides corresponding to such sequences.

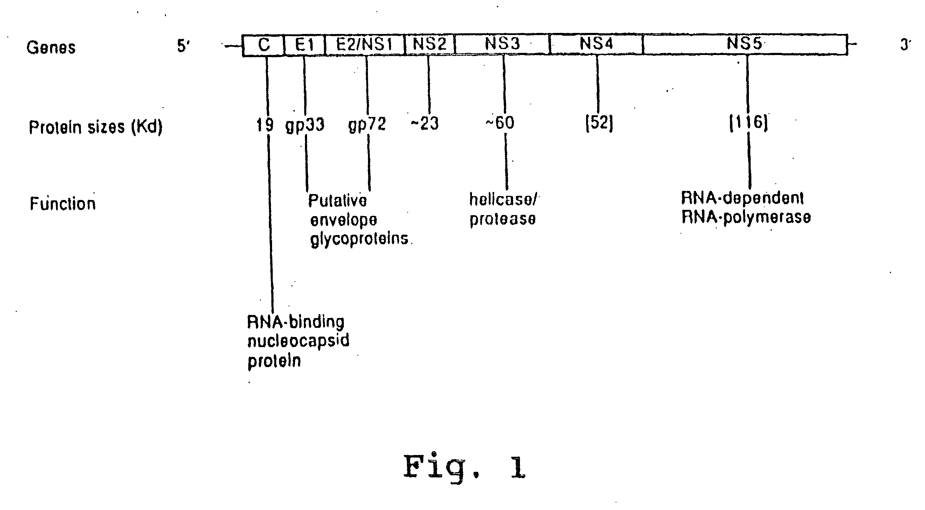

The nucleic acid sequences described define genotypes of HCV with respect to four regions of the viral genome. Figure 1 depicts schematically the organization of HCV. The four regions of particular interest are the NS5 region, the envelope 1 region, the 5'UT region and the core region.

The sequences set forth in the present application as sequences numbered 33-51 suggest at least three genotypes in the 5'UT region of HCV. Sequences numbered 33-51 are depicted in Figure 4 as well as in the Sequence Listing. Each sequence numbered 33-51 is derived from the nucleic acid having 252 nucleotides from the 5'UT region, although sequences 50 and 51 are somewhat shorter at approximately 180 nucleotides.

The first 5'UT genotype (GI) is exemplified by the sequences within sequences numbered 33-38. A second 5'UT genotype (GII) is exemplified by the sequences within sequences numbered 39-45. A third 5'UT genotype (GIII) is exemplified by the sequences within sequences numbered 46-47. A fourth 5'UT genotype (GIV) is exemplified by sequences within sequences humbered 48 and 49. A fifth 5'UT genotype (GV) is exemplified by sequences within sequences numbered 50 and 51.

The various genotypes described with respect to each region are consistent. That is, HCV having features of the first genotype with respect to the NS5 region will substantially conform to features of the first genotype of the envelope 1 region, the 5' UT region and the core region.

Nucleic acid isolated or synthesized in accordance with the sequences set forth in sequence numbers 1-66 are useful as probes, primers, capture ligands and anti-sense agents. As probes, primers, capture ligands and anti-sense agents,the nucleic acid wil normally comprise approximately eight or more nucleotides for specificity as well as the ability to form stable hybridization products.

### Probes

A nucleic acid isolated or synthesized in accordance with a sequence defining a particular genotype of a region of the HCV genome can be used as a probe to detect such genotype or used in combination with other nucleic acid probes to detect substantially all genotypes of HCV.

With the sequence information set forth in the present application, sequences of eight or more nucleotides are identified which provide the desired inclusivity and exclusivity with respect to various genotypes within HCV. and extraneous nucleic acid sequences likely to be encountered during hybridization conditions.

Individuals skilled in the art will readily recognize that the nucleic acid sequences, for use as probes, can be provided with a label to facilitate detection of a hybridization product.

### Capture Ligand

For use as a capture ligand, the nucleic acid selected in the manner described above with respect to probes, can be readily associated with supports. The manner in which nucleic acid is associated with supports is well known. Nucleic acid having sequences corresponding to a sequence within sequences numbered 1-66 have utility to separate viral nucleic acid of one genotype from the nucleic acid of HCV of a different genotype. Nucleic acid isolated or synthesized in accordance with sequences within sequences numbered 1-66, used in combinations, have utility to capture substantially all nucleic acid of all HCV genotypes.

### Primers

Nucleic acid isolated or synthesized in accordance with the sequences described herein have utility as primers for the amplification of HCV sequences. With respect to polymerase chain reaction (PCR) techniques, nucleic acid sequences of eight or more nucleotides corresponding to one or more sequences of sequences numbered 1-66 have utility in conjunction with suitable enzymes and reagents to create copies of the viral nucleic acid. A plurality of primers having different sequences corresponding to more than one genotype can be used to create copies of viral nucleic acid for such genotypes.

The copies can be used in diagnostic assays to detect HCV virus. The copies can also be incorporated into cloning and expression vectors to generate polypeptides corresponding to the nucleic acid synthesized by PCR, as will be described in greater detail below.

### Anti-sense

Nucleic acid isolated or synthesized in accordance with the sequences described herein have utility as anti-sense genes to prevent the expression of HCV.

Nucleic acid corresponding to a genotype of HCV is loaded into a suitable carrier such as a liposome for introduction into a cell infected vith HCV. A nucleic acid having eight or more nucleotides is capable of binding to viral nucleic acid or viral messenger RNA. Preferably, the anti-sense nucleic acid is comprised of 30 or more nucleotides to provide necessary stability of a hybridization product of viral-nucleic acid or viral messenger RNA. Methods for loading anti-sense nucleic acid is known in the art as exemplified by U.S. Patent: 4,241,046 issued December 23, 1980 to Papahadjopoulos et al.

### Peptide Synthesis

Nucleic acid isolated or synthesized in accordance with the sequences described herein have utility to generate peptides. The sequences exemplified by sequences numbered 1-32 and 52-66 can be cloned into suitable vectors or used to isolate nucleic acid. The isolated nucleic acid is combined with suitable DNA linkers and cloned into a suitable vector. The vector can be used to transform, a suitable host organism such as E. coli and the peptide encoded by the sequences isolated.

Molecular cloning techniques are described in the text Molecular Cloning: A Laboratory Manual, Maniatis et al., Coldspring Harbor Laboratory (1982).

The isolated peptide bas utility as an antigenic substance for the has utility as an antigenic antibodies directed to the particular genotype-of HCV.

### Vaccines and Antibodies

The peptide materials of the present invention have utility for development of antibodies and vaccines.

The availability of sequences. or nucleotide sequences derived therefrom (including segments and modifications of the sequence), permits the construction of expression vectors encoding antigenically active regions of the peptide encoded in either strand. The antigenically active regions may be derived from the NS5 region, envelope 1 regions, and the core region.

Fragments encoding the desired peptides are derived from the cDNA clones using conventional restriction digestion or by synthetic methods, and are ligated into vectors which may, for example, contain portions of fusion sequences such as beta galactosidase or superoxide dismutase (SOD), preferably SOD. Methods and vectors which are useful for the production of polypeptides which contain fusion sequences of SOD are described in European Patent Office Publication number 0196056, published October 1, 1986.

Any desired portion of the HCV cDNA containing an open reading frame, in either sense strand, can be obtained as a recombinant peptide, such as a mature or fusion protein; alternatively, a peptide encoded in the cDNA can be provided by chemical synthesis.

The DNA encoding the desired peptide, whether in fused or mature form. and whether or not containing a signal sequence to permit secretion, may be ligated into expression vectors suitable for any convenient host. Both eukaryotic and prokaryotic host systems are presently used in forming recombinant peptides. The peptide is then isolated from lysed cells or from the culture medium and purified to the extent needed for its intended use. Purification may be by techniques known in the art, for example, differential extraction. salt fractionation, chromatography on ion exchange resins, affinity chromatography, centrifugation, and the like. See, for example, Methods in Enzymology for a variety of methods for purifying proteins. Such peptides can be used as diagnostics, or those which give rise to neutralizing antibodies may be formulated into vaccines. Antibodies raised against these peptides can also be used as diagnostics, or for passive immunotherapy or for isolating and identifying HCV.

An antigenic region of a peptide is generally relatively small-typically 8 to 10 amino acids or less in length. Fragments of as few as 5 amino acids may characterize an antigenic region. These segments may correspond to NS5 region, envelope 1 region, and the core region of the HCV genome. The 5'UT region is not known to be translated. Accordingly, using the cDNAs of such regions, DNAs encoding short segments of HCV peptides corresponding to such regions can be expressed recombinantly either as fusion proteins, or as isolated peptides. In addition, short amino acid sequences can be conveniently obtained by chemical synthesis. In instances wherein the synthesized peptide is correctly configured so as to provide the correct epitope, but is too small to be immunogenic, the peptide may be linked to a suitable carrier.

A number of techniques for obtaining such linkage are known in the art, including the formation of disulfide linkages using N-succinimidyl-3-(2-pyridylthio)propionate (SPDP) and succinimidyl 4-(N-maleimido-methyl)cyclohexane-1-carboxylate (SMCC) obtained from Pierce Company, gockford, Illinois, (if the peptide lacks a sulfhydryl group, this can be provided by addition of a cysteine residue). These reagents create a disulfide linkage between themselves and peptide cysteine residues on one protein and an amide linkage through the epsilon-amino on a lysine, or other free amino group in the other. A variety of such disulfide/amide-forming agents are Known. See, for example, Immun Rev (1982) 62:185. Other bifunctional coupling agents form a thioether rather than a disulfide linkage. Many of these thio-ether-forming agents are commercially available and include reactive esters of 6-maleimidocaprioc acid, 2-bromoacetic acid. 2-iodoacetic acid, 4-N-maleimido-methyl)cyclohexane-1-carboxylic acid, and the like. The carboxyl groups can be activated by combining them with succinimide or 1-hydroxyl-2 nitro-4-sulfonic acid, sodium salt. Additional methods of coupling antigens employs the rotavirus/"binding peptide" system described in EPO Pub. No. 259, 149, the disclosure of which is incorporated herein by reference. The foregoing list is not meant to be exhaustive, and modifications of the named compounds can clearly be used.

Any carrier may be used which does not itself induce the production of antibodies harmful to the host. Suitable carriers are typically large, slowly metabolized macromolecules such as proteins; polysaccharides, such as latex functionalized Sepharose, agarose, cellulose, cellulose beads and the like; polymeric amino acids, such as polyglutamic acid, polylysine, and the like; amino acid copolymers; and inactive virus particles. Especially useful protein substrates are serum albumins, keyhole limpet hemocyanin, immunoglobulin molecules, thyroglobulin, ovalbumin, tetanus toxoid and other proteins well known to those skilled in the art.

Hepatitis surface antigen (HBSAg) has been shown to be formed and assembled into particles in S: cerevisiae (P. Valenzuela et al. (1982)), as well as in, for example, mammalian cells (P. Valenzuela et al. 1984)). The formation of such particles has been shown to enhance the immunogenicity of the monomer subunit. The constructs may also include the immunodominant epitope of HBSAg, comprising the 55 amino acids of the presurface (pre-S) region. Neurath et al. (1984). Constructs of the pre-S-HBSAg particle expressible in yeast are disclosed in EPO 174,444, published March 19, 1986; hybrids including heterologous viral sequences for yeast expression are disclosed in EPO 175,261, published March 26, 1966. These constructs may also be expressed in mammalian cells such as Chinese hamster ovary (CHO) cells using an SV40-dihydrofolate reductase vector (Michelle et al. (1984)).

In addition, portions of the particle-forming protein coding sequence may be replaced with codons encoding an HCV epitope. In this replacement, regions which are not required to mediate the aggregation of the units to form immunogenic particles in yeast of mammals can be deleted, thus eliminating additional HBV antigenic sites from competition with the HCV epitope.

### I. Detection of HCV RNA from Serum

RNA was extracted from serum using guanidinium salt, phenol and chloroform according to the instructions of the kit manufacturer (RNAzol™ B kit, Cinna/Biotecx). Extracted RNA was precipitated with isopropanol and washed with ethanol. A total of 25 µl serum was processed for RNA isolation, and the purified RNA was resuspended in 5 µl diethyl pyrocarbonate treated water for subsequent cDNA synthesis.

### II. cDNA Synthesis and Polymerase Chain Reaction (PCR) Amplification

Table 1 lists the sequence and position (with reference to HCVl) of all the PCR primers and probes used in these examples. Letter designations for nucleotides are consistent with 37 C.F.R. §§1.821-1.825. Thus, the letters A, C, G, T, and U are used in the ordinary sense of adenine, cytosine, guanine. thymine, and uracil. The letter M means A or C; R means A or G; W means A or T/U; S means C or G; Y means C or T/U; K means G or T/U; V means A or C or G, not T/U; H means A or C or T/U, not G; D means A or G or T/U, not C; B means C or G or T/U, not A; N means (A or C or G or T/U) or (unknown or other). Table 1 is set forth below:

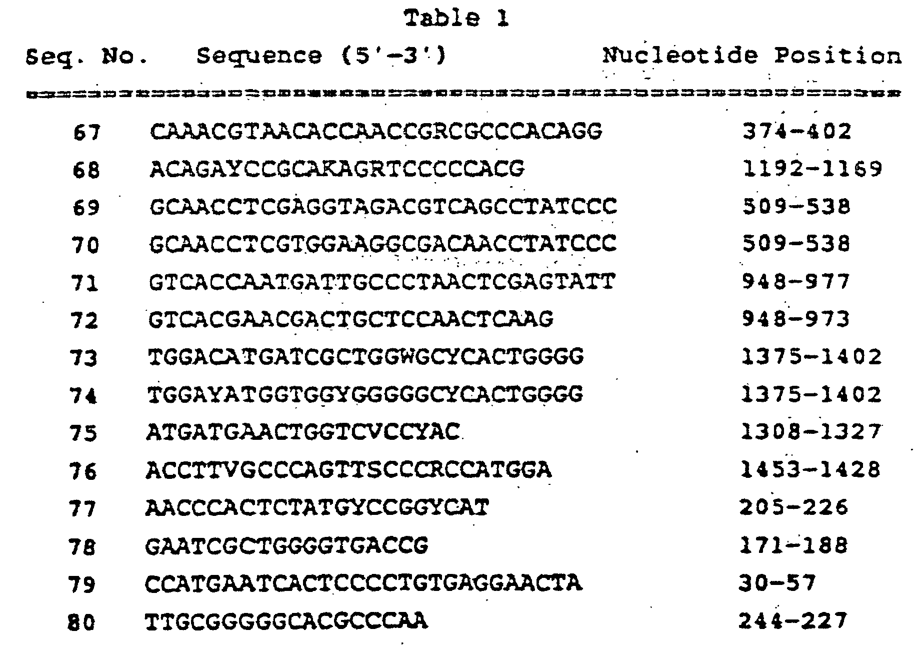

For cDNA synthesis and PCR amplification, a protocol developed by Perkin-Elmer/Cetus (GeneAmp® RNA PCR kit) was used. Both random hexamer and primers with specific complementary sequences to HCV were employed to prime the reverse transcription (RT) reaction. All processes, except for adding and mixing reaction components, were performed in a thermal cycler (MJ Research. Inc.). The first strand cDNA. synthesis reaction was inactivated at 99°C for 5 min, and then cooled at 50°C for 5 min before adding reaction components for subsequent amplification. After an initial 5 cycles of 97°C for 1 min, 50°C for 2 min, and 72°C for 3 min, 30 cycles of 94°C for 1 min, 55°C for 2 min, and 72°C for 3 min followed, and then a final 7 min of elongation at 72°C.

For the genotyping analysis, sequences 67 and 68 were used as primers in the PCR reaction. These primers amplify a segment corresponding to the core and envelope regions. After amplification, the reaction products were separated on an agarose gel and then transferred to a nylon membrane. The immobilized reaction products were allowed to hybridize with. a ³²P-labelled nucleic acid corresponding to either Genotype I (core or envelope 1) or Genotype II (core or envelope 1). Nucleic acid corresponding-to Genotype I comprised sequences numbered 69 (cote). 71 (envelope), and 73 (envelope). Nucleic acid corresponding to. Genotype II comprised sequences numbered 70 (core), 72 (envelope), and 74 (envelope).

The Genotype I probes only hybridized to the product amplified from isolates which had Genotype I sequence. Similarly, Genotype II probes only hybridized to the product amplified from isolates which had Genotype II sequence.

In another experiment, PCR products were generated using sequences 79 and 80. The products were analyzed as described above except Sequence No. 73 was used to detect Genotype I, Sequence No. 74 was used to detect Genotype II, Sequence No. 77 (5'UT) was used to detect Genotype III, and Sequence No. 78 (5'UT) was used to detect Genotype IV. Each sequence hybridized in a genotype specific manner.

### III. Detection of HCV GI-GIV using a sandwich hybridization essay for HCV RNA

An amplified solution phase nucleic acid sandwich hybridization assay format is described in this example. The assay format employs several nucleic acid probes to effect capture and detection. A capture probe nucleic acid is capable of associating a complementary probe bound to a solid support and HCV nucleic acid to effect capture. A detection probe nucleic acid has a first segment (A) that binds to HCV nucleic acid and a second segment (B) that hybridizes to a second amplifier nucleic acid. The amplifier nucleic acid has a first segment (B*) that hybridizes to segment (B) of the probe nucleic acid and also comprises fifteen iterations of a segment (C). Segment C of the amplifier nucleic acid is capable of hybridizing to three labeled nucleic acids.

Nucleic acid sequences which correspond to nucleotide sequences of the envelope 1 gene of Group I HCV isolates are set forth in sequences numbered 81-99. Table 2 sets forth the area of the HCV genome to which the nucleic acid sequences correspond and a preferred use of the sequences.

**Table 2**

| Probe Type | Sequence No. | Complement of Nucleotide Numbers |
|---|---|---|
| Label | 81 | 879-911 |
| Label | 82 | 912-944 |
| Capture | 83 | 945-977 |
| Label | 84 | 978-1010 |
| Label | 85 | 1011-1043 |
| Label | 86 | 1044-1076 |
| Label | 87 | 1077-1109 |
| Capture | 88 | 1110-1142 |
| Label | 89 | 1143-1175 |
| Label | 90 | 1176-1208 |
| Label | 91 | 1209-1241 |
| Label | 92 | 1242=1274 |
| Capture . | 93 | 1275-1307 |
| Label | 94 | 1308-1340 |
| Label | 95 | 1341-1373 |
| Label | 96 | 1374-1406 |
| Label | 97 | 1407-1439 |
| Capture | 98 | 1440-1472 |
| Label | 99 | 1473-1505 |

Nucleic acid sequences which correspond to nucleotide sequences of the envelope 1 gene of Group II HCV isolates are set forth in sequences 100-118. Table 3 sets forth the area of the HCV genome to which the nucleic acid corresponds and the preferred use of the sequences.

**Table 3**

| Probe Type | Sequence No. | Complement of Nucleotide Numbers |
|---|---|---|
| Label | 100 | 879-911 |
| Label | 101 | 912-944 |
| Capture | 102 | 945-977 |
| Label | 103 | 978-1010 |
| Label | 104 | 1011-1043 |
| Label | 105 | 1044-1076 |
| Label | 106 | 1077-1109 |
| Capture | 107 | 1110-1142 |
| Label | 108 | 1143-1175 |
| Label | 109 | 1176-1208 |
| Label | 110 | 1209-1241 |
| Label | 111 | 1242-1274 |
| Capture | 112 | 1275-1307 |
| Label | 113 | 1308-1340 |
| Label | 114 | 1341-1373 |
| Label | 115 | 1374-1406 |
| Label | 116 | 1407-1439 |
| Capture | 117 | 1440-1472 |
| Label | 118 | 1473-1505 |

Nucleic acid sequences which correspond to nucleotide sequences in the C gene and the 5'U,T region are set forth in sequences 119-145. Table 4 identifies the sequence with a preferred use.

**Table 4**

| Probe Type | Sequence No. |
|---|---|
| Capture | 119 |
| Label | 120 |
| Label | 121 |
| Label | 122 |
| Capture | 123 |
| Label | 124 |
| Label | 125 |
| Label | 126 |
| Capture | 127 |
| Label | 128 |
| Label | 129 |
| Label | 130 |
| Capture | 131 |
| Label | 132 |
| Label | 133 |
| Label | 134 |
| Label | 135 |
| Capture | 136 |
| Label | 137 |
| Label | 138 |
| Label | 139 |
| Capture | 140 |
| Label | 141 |
| Label | 142 |
| Label | 143 |
| Capture | 144 |
| Label | 145 |

The detection and capture probe HCV-specific segments, and their respective names as used in this assay were as follows.
Capture sequences are sequences numbered 119-122 and 141-144.
Detection sequences are sequences numbered 119-140.

Each detection sequence contained, in addition to the sequences substantially complementary to the HCV sequences, a 5' extension (B) which extension (B) is complementary to a segment of the second amplifier nucleic acid. The extension (B) sequence is identified in the Sequence Listing as Sequence No. 146. and is reproduced below.

Each capture sequence contained, in addition to the sequences substantially complementary to HCV sequences, a sequence complementary to DNA bound to a solid phase. The sequence complementary to DNA bound to a solid support was carried downstream from the capture sequence. The sequence complementary to the DNA bound to the support is set forth as Sequence No. 147 and is reproduced below.

Microtiter plates were prepared as follows. White Microlite 1 Removawell strips (polystyrene microtiter plates, 96 veils/plate) were purchased from Dynatech Inc.

Each well was filled with 200 µl 1 N HC1 and incubated at room temperature for 15-20 min. The plates were then washed 4 times with 1X PBS and the wells aspirated to remove liquid. The wells were then filled vith 200 µl 1 N NaOH and incubated at room temperature for 15-20 min. The plates were again washed 4 times with 1X PBS and the wells aspirated to remove liquid.

Poly(phe-lys) was purchased from Sigma Chemicals, Inc. This polypeptide has a 1:1 molar ratio of phe:lys and an average m.w. of 47,900 gm/mole. It has an average length of 309 amino acids and contains 155 amines/mole. A 1 mg/ml solution of the polypeptide was mixed with 2M NaCl/1X PBS to a final concentration of 0.1 mg/ml (pH 6.0). A volume of 200 µl of this solution was added to each well. The plate was wrapped in plastic to prevent drying and incubated at 30°C overnight. The plate was then washed 4 times with 1X PBS and the wells aspirated to remove liquid.

The following procedure was used to couple the nucleic acid, a complementary sequence to Sequence No. 147, to the plates, hereinafter referred to as immobilized nucleic acid. Synthesis of immobilized nucleic acid having a sequence complementary to Sequence No. 133 was described in EPA-883096976. A quantity of 20 mg disuccinimidyl suberate was dissolved in 300 µl dimethyl formamide (DMF). A quantity of 26 OD₂₆₀ units of immobilized nucleic acid was added to 100 µl coupling buffer (50 mM sodium phosphate, pH 7.8). The coupling mixture was then added to the DSS-DMF solution and stirred with a magnetic stirrer for 30 min. An NAP-25 column was equilibrated with 10 nM sodium phosphate, pH 6.5. The coupling mixture DSS-DMF solution was added to 2 ml 10 mM sodium phosphate, pH 6.5, at 4°C. The mixture was vortexed to mix and loaded onto the equilibrated NAP-25 column. DSS-activated immobilized nucleic acid DNA was eluted from the column with 3.5 ml 10 mM sodium phosphate, pH 6.5. A quantity of 5.6 OD₂₆₀ units of eluted DSS-activated immobilized nucleic acid DNA was added to 1500 ml 50 mM sodium phosphate, pH 7.8. A volume of 50 µl of this solution was added to each well and the plates ware incubated overnight. The plate was then washed 4 times with 1X PBS and the wells aspirated to remove liquid.

Final stripping of plates was accomplished as follows. A volume of 200 µl of 0.2N NaOH containing 0.5% (w/v) SDS was added to each well. The plate was wrapped in plastic and incubated at 65°C for 60 min. The plate was then washed 4 times with 1X PBS and the wells aspirated to remove liquid. The stripped plate was stored with desiccant beads at 2-8'C.

Serum samples to be assayed were analyzed using PCR followed by sequence analysis to determine the genotype.

Sample preparation consisted of delivering 50 µl of the serum sample and 150 µl P-K Buffer (2- mg/ml proteinase K in 53 mM Tris-HC1, pH 8.0/0.6 M NaCl/0.06 M sodium citrate/8 mM EDTA, pH 8.0/1. 3%SDS/16µg/ml sonicated salmon sperm DNA/7% formamide/50 fmoles capture probes/160 fmoles detection probes) to each well. Plates were agitated to mix the contents in the well, covered and incubated for 16 hr at 62°C.

After a further 10 minute period at room temperature, the contents of each well were aspirated to remove all fluid, and the wells washed 2X with washing buffer (0.1% SDS/0.015 M MaCl/ 0.0015 M sodium citrate). The amplifier nucleic acid was then added to each well (50 µl of 0.7 fmole/µl solution in 0..48 M NaCl/0.048 M sodium citrate/ 0.1% SDS/0.5% "blocking reagent" (Boehringer Mannheim, catalog No. 1096 176)). After covering the plates and agitating to mix the contents in the wells, the plates were incubated for 30 min. at 52°C.

After a further 10 min period at room temperature, the wells were washed as described above.

Alkaline phosphatase label nucleic acid, disclosed in EP 883096976, was then added to each well (50 (µl/well of 2.66 fmoles/µl). After incubation at 52°C for 15 min., and 10 min. at room temperature, the wells were washed twice as above and then 3X with 0.015 M NaCl/0.0015 M sodium citrate.

An enzyme-triggered dioxetane (Schaap et al., Tet. Lett. (1987) 28:1159-1162 and EPA Pub. No. 0254051), obtained from Lumigen, Inc. , was employed. A quantity of 50 µl Lumiphos 530 (Lumigen) was added to each well. The wells were tapped lightly so that the reagent would fall to the bottom and gently swirled to distribute the reagent evenly over the bottom. The wells were covered and incubated at 37°C for 20-40 min.

Plates were then read on a Dynatech ML 1000 luminometer. Output was given as the full integral of the light produced during the reaction.

The assay positively detected each of the serum samples, regardless of genotype.

### IV. Expression of the Polypeptide Encoded in Sequences Defined by Differing Genotypes

HCV polypeptides encoded by a sequence within sequences 1-66 are expressed as a fusion polypeptide with superoxide dismutase (SOD). A cDNA carrying such sequences is subcloned into the expression vector pSODcf1 (Steimer et al. 1986)).

First, DNA isolated from pSODcfl is treated with BamHI and EcoRI, and the following linker was ligated into the linear DNA created by the restriction enzymes: After cloning, the plasmid containing the insert is isolated.

Plasmid containing the insert is restricted with EcoRI. The HCV cDNA is ligated into this EcoRI linearized plasmid DNA. The DNA mixture is used to transform E. coli strain D1210 (Sadler et al. (1980)). Polypeptides are isolated on gels.

### V. Antigenicity of Polypeptides

The antigenicity of polypeptides formed in Section IV is evaluated in the following manner. Polyethylene pins arranged on a block in an 8 12 array (Coselco Mimetopes, Victoria, Australia) are prepared by placing the pins in a bath (20% v/v piperidine in dimethylformamide (DMF)) for 30 minutes at room temperature. The pins are removed, washed in DMF for 5 minutes, then washed in methanol four times (2. min/wash). The.pins are allowed to air dry for at least 10 minutes, then washed a final time in DMF (5Win). 1-Hydroxybenzotriazole (HOBt, 367 mg) is dissolved in DMF (80 µL) for use in coupling Fmoc-protected polypeptides prepared in Section IV.

The protected amino acids are placed in micro-titer plate wells with Host, and the pin block placed over the plate, immersing the pins in the wells. The assembly is then sealed in a plastic bag and allowed to react at 25°C for 18 hours to couple the first amino acids to the pins. The block is then removed, and the pins washed with DMF (2 min.), MeOH (4 x, 2 min.), and again with DMF (2 min.) to clean and deprotect the bound amino acids. The procedure is repeated for each additional amino acid coupled, until all octamers are prepared.

The free N-termini are then acetylated to compensate for the free amide, as most cf the epitopes are not found at the N-terminus and thus would not have the associated positive charge. Acetylation is accomplished by filling the wells of a microtiter plate with DMF/acetic anhydride/triethylamine (5:2:1 v/v/v) and allowing the pins to react in the wells for 90 minutes at 20°C. The pins are then washed with DMF (2 min.) and MeOH (4 x, 2 min.), and air dried for at least 10 minutes.

The side chain protecting groups are removed by treating the pins with trifluoroacetic acid/phenol/dithioethane (95:2.5:1.5, v/v/v) in polypropylene bags for 4 hours at room temperature. The pins are then washed in dichloromethane (2 x, 2 min.), 5% di-isopropylethylamine/dichloromethane (2 x, 5 min.), dichloromethane (5 min.) , and air-dried for at least 10 minutes. The pins are then washed in water (2 min.), MeOH (18 hours), dried in vacuo, and stored in sealed plastic bags over silica gel- IV.B.15.b Assay of Peptides.

Octamer-bearing pins are treated by sonicating for 30 minutes in a disruption buffer (1% sodium dodecylsulfate, 0.1% 2-mercaptoethanol, 0.1 M NaH2PO4) at 60°C. The pins are then immersed several times in water (60°C), followed by boiling MeOH (2 min.), and allowed to air dry.

The pins are then precoated for 1 hour at 25°C in microtiter wells containing 200 µL blocking buffer (1% ovalbumin, 1% BSA, 0.1% Tween, and 0.05% NaN3 in PBS). with agitation. The pins are then immersed in microtiter wells containing 175 µL antisera obtained from human patients diagnosed as having HCV and allowed to incubate at 4°C overnight. The formation of a complex between polyclonal antibodies of the serum and the polypeptide initiates that the peptides give rise to an immune response in vivo. Such peptides are candidates for the development of vaccines.

Thus, this invention has been described and illustrated. It will be apparent to those skilled in the art that many variations and modifications can be made without. departing from the purview of the appended claims and without departing from the teaching and scope of the present invention.

## Claims (Claims for the following Contracting State(s): AT, BE, CH, LI, DE, DK, FR, GB, IT, LU, MC, NL, SE)

1. A non-naturally occurring nucleic acid comprising a non-HCV-1 nucleotide sequence consisting of from 8 to 252 contiguous nucleotides which are fully homologous to or fully complementary to a nucleotide sequence from the 5'UT region of a hepatitis C virus and selected from a sequence within SEQ ID Nos 34 to 49 and wherein said sequence is not HCV-J1 or HCV-J4.

2. A non-naturally occurring nucleic acid comprising a non-HCV-1 nucleotide sequence consisting of from 8 to 180 contiguous nucleotides which are fully homologous to or fully complementary to a nucleotide sequence from the 5'UT region of a hepatitis C virus and selected from a sequence within SEQ ID NOs 50 and 51 and wherein said sequence is not HCV-J1 or HCV-J4.

3. A nucleic acid according to claim 1 corresponding to a nucleotide sequence within the 5' UT region of a first hepatitis C virus genotype wherein said non-HCV-1 nucleotide sequence is selected from a sequence within SEQ ID Nos 34 to 38.

4. A nucleic acid according to claim 1 within the 5' UT region of a second hepatitis C virus genotype wherein said non-HCV-1 nucleotide sequence is selected from a sequence within SEQ ID Nos 39 to 45.

5. A nucleic acid according to claim 1 within the 5' UT region of a third hepatitis C virus genotype wherein said non-HCV-1 nucleotide sequence is selected from a sequence within SEQ ID Nos 46 and 47.

6. A nucleic acid according to claim 1 within the 5' UT region of a fourth hepatitis C virus genotype wherein said non-HCV-1 nucleotide sequence is selected from a sequence within SEQ ID Nos 48 and 49.

7. A nucleic acid according to any one of claims 1 to 6 which is capable of priming a reaction for the synthesis of nucleic acid to form a nucleic acid having a nucleotide sequence corresponding to hepatitis C virus.

8. A nucleic acid according to any one of claims 1 to 6 having a label means for detecting a hybridization product.

9. A nucleic acid according to any one of claims 1 to 6 having support means for separating a hybridization product from solution.

10. A nucleic acid according to any one of claims 1 to 6 which is capable of preventing the transcription or translation of viral nucleic acid.

11. A method of forming a hybridization product which a hepatitis C virus nucleic acid comprise the following steps:
(a) placing a non-naturally occurring nucleic acid according to any one of claims 1 to 9 under conditions in which hybridization conditions can be imposed, said nucleic acid being capable of forming a hybridization product with said hepatitis C virus nucleic acid under hybridization conditions; and
(b) imposing hybridization conditions to form a hybridization product in the presence of hepatitis C virus nucleic acid.

12. A method of detecting one or more genotypes of hepatitis C virus comprising the following steps:
(a) placing a non-naturally occurring nucleic acid according to any one of claims 1 to 9 under conditions in which hybridization conditions can be imposed,
(b) imposing hybridization conditions to form a hybridization product in the presence of a hepatitis C virus nucleic acid; and
(c) monitoring the non-naturally occurring nucleic acid for the formation of a hybridization product, which hybridization product is indicative of the presence of the genotype of hepatitis C virus.

## Claims (Claims for the following Contracting State(s): GR, ES)

1. A method of forming a hybridization product with a hepatitis C virus nucleic acid comprising the following steps:
(a) placing a non-naturally occurring nucleic acid comprising a non-HCV-1 nucleotide sequence consisting of from 8 to 252 contiguous nucleotides which are fully homogolous to or fully complementary to a nucleotide sequence from the 5' UT region of a hepatitis C virus and selected from a sequence within SEQ ID Nos 34 to 49 and wherein said sequence is not HCV-J1 or HCV-J4, under conditions in which hybridization conditions can be imposed, said nucleic acid being capable of forming a hybridization product with said hepatitis C virus nucleic acid under hybridization conditions; and
(b) imposing hybridization conditions to form a hybridization product in the presence of hepatitis C virus nucleic acid.

2. A method of forming a hybridization product with a hepatitis C virus nucleic acid comprising the following steps:
(a) placing a non-naturally occurring nucleic acid comprising a non-HCV-1 nucleotide sequence consisting of from 8 to 180 contiguous nucleotides which are fully homogolous to or fully complementary to a nucleotide sequence from the 5'UT region of a hepatitis C virus and selected from a sequence within SEQ ID Nos 50 to 51 and wherein said sequence is not HCV-J1 or HCV-J4, under conditions in which hybridization conditions can be imposed, said nucleic acid being capable of forming a hybridization product with said hepatitis C virus nucleic acid under hybridization conditions; and
(b) imposing hybridization conditions to form a hybridization product in the presence of hepatitis C virus nucleic acid.

3. The method of claim 1, wherein said nucleic acid corresponds to a nucleotide sequence within the 5'UT region of a first hepatitis C virus genotype, and wherein said non-HCV-1 nucleotide sequence is selected from a sequence within SEQ ID Nos 34 to 38.

4. The method of claim 1, wherein said nucleic acid is within the 5'UT region of a second hepatitis C virus genotype and wherein said non-HCV-1 nucleotide sequence is Selected from a sequence within SEQ ID Nos 39 to 45.

5. The method of claim 1, wherein said nucleic acid is within the 5'UT region of a third hepatitis C virus genotype and wherein said non-HCV-1 nucleotide sequence is selected from a sequence within SEQ ID Nos 46 and 47.

6. The method of claim 1, wherein said nucleic acid is within the 5'UT region of a fourth hepatitis C virus genotype and wherein said non-HCV-1 nucleotide sequence is selected from a sequence within SEQ ID Nos 48 and 49.

7. The method of any of claims 1 to 6, wherein said nucleic acid is capable of priming a reaction for the synthesis of nucleic acid to form a nucleic acid having a nucleotide sequence corresponding to hepatitis C virus.

8. The method of any of claims 1 to 6, wherein said nucleic acid has a label means for detecting a hybridization product.

9. The method of any of claims 1 to 6, wherein said nucleic acid has support means for separating a hybridization product from solution.

10. A method of detecting one or more genotypes of hepatitis C virus comprising the following steps:
(a) placing a non-naturally occurring nucleic acid comprising a non-HCV-1 nucleotide sequence consisting of from 8 to 2'52 contiguous nucleotides which are fully homogolous to or fully complementary to a nucleotide sequence from the 5'UT region of a hepatitis C virus and selected from a sequence within SEQ ID Nos 34 to 49 and wherein said sequence is not HCV-J1 or HCV-J4, under conditions in which hybridization conditions can be imposed,
(b) imposing hybridization conditions to form a hybridization product in the presence of hepatitis C virus nucleic acid; and
(c) monitoring the non-naturally occurring nucleic acid for the formation of a hybridization product, which hybridization product is indicative of the presence of the genotype of hepatitis C virus.

11. A method of detecting one or more genotypes of hepatitis C virus comprising the following steps:
(a) placing a non-naturally occurring nucleic acid comprising a non-HCV-1 nucleotide sequence consisting of from 8 to 180 contiguous nucleotides which are fully homogolous to or fully complementary to a nucleotide sequence from the 5'UT region of a hepatitis C virus and selected from a sequence within SEQ ID Nos 50 and 51 and wherein said sequence is not HCV-J1 or HCV-J4, under conditions in which hybridization conditions can be imposed,
(b) imposing hybridization conditions to form a hybridization product in the presence of hepatitis C virus nucleic acid; and
(c) monitoring the non-naturally occurring nucleic acid for the formation of a hybridization product, which hybridization product is indicative of the presence of the genotype of hepatitis C virus.

12. The method of claim 10, wherein said nucleic acid corresponds to a nucleotide sequence within the 5'UT region of a firsc hepatitis C virus genotype, and wherein said non-HOV-1 nucleotide sequence is selected from a sequence within SEQ ID Nos 34 to 38.

13. The method of claim 10, wherein said nucleic acid is within the 5' UT region of a second hepatitis C virus genotype and wherein said non-HCV-1 nucleotids sequence is selected from a sequence within SEQ ID Nos 39 to 45.

14. The method of claim 10, wherein said nucleic acid is within the 5' UT region of a third hepatitis C virus genotype and wherein said non-HCV-1 nucleotide sequence is selected from a sequence within SEQ ID Nos 46 and 47.

15. The method of claim 10, wherein said nucleic acid is within the 5'UT region of a fourth hepatitis C virus genotype and wherein said non-HCV-1 nucleotide sequence is selected from a sequence within SEQ ID Nos 48 and 49.

16. The method of any of claims 10 to 15, wherein said nucleic acid is capable of priming a reaction for the synthesis of nucleic acid to form a nucleic acid having a nucleotide sequence corresponding to hepatitis C virus.

17. The method of any of claims 10 to 15, wherein said nucleic acid has a label means for detecting a hybridization product.

18. The method of any of claims 10 to 15, wherein said nucleic acid has support means for separating a hybridization product from solution.

## Patentansprüche (Patentansprüche für folgende(n) Vertragsstaat(en): AT, BE, CH, LI, DE, DK, FR, GB, IT, LU, MC, NL, SE)

1. Nicht natürlich vorkommende Nukleinsäure, die eine nicht-SE HCV-1 Nukleotidsequenz umfasst, die aus 8 bis 252 aufeinanderfolgenden Nukleotiden besteht, die vollständig homolog oder vollständig komplementär zu einer Nukleotidsequenz aus der 5'UT-Region eines Hepatitis C Virus sind, und aus einer Sequenz innerhalb der SEQ ID Nrn. 34 bis 49 ausgewählt ist, und wobei die genannte Sequenz weder HCV-J1 noch HCV-J4 ist.

2. Nicht natürlich vorkommende Nukleinsäure, die eine nicht-HCV-1 Nukleotidsequenz umfasst, die aus 8 bis 180 aufeinanderfolgenden Nukleotiden besteht, die vollständig homolog oder vollständig komplementär zu einer Nukleotidsequenz aus der 5'UT-Region eines Hepatits C Virus ist und aus einer Sequenz innerhalb der SEQ ID Nrn. 50 und 51 ausgewählt ist, und wobei die genannte Sequenz weder HCV-J1 noch HCV-J4 ist.

3. Nukleinsäure nach Anspruch 1, entsprechend einer Nukleotidsequenz innerhalb der 5'UT-Region eines ersten Hepatitis C Virengenotyps, wobei die genannte nicht-HCV-1 Nukleotidsequenz aus einer Sequenz innerhalb der SEQ ID Nrn. 34 bis 38 ausgewählt ist.

4. Nukleinsäure nach Anspruch 1, innerhalb der 5'UT-Region eines zweiten Hepatitis C Virengenotyps, wobei die genannte nicht-HCV-1 Nukleotidsequenz aus einer Sequenz innerhalb der SEQ ID Nrn. 39 bis 45 ausgewählt ist.

5. Nukleinsäure nach Anspruch 1, innerhalb der 5'UT-Region eines dritten Hepatitis C Virengenotyps, wobei die genannte nicht-HCV-1 Nukleotidsequenz aus einer Sequenz innerhalb der SEQ ID Nrn. 46 und 47 ausgewählt ist.

6. Nukleinsäure nach Anspruch 1, innerhalb der 5'UT-Region eines vierten Hepatitis C Virengenotyps, wobei die genannte nicht-HCV-1 Nukleotidsequenz aus einer Sequenz innerhalb der SEQ ID Nrn. 48 und 49 ausgewählt ist.

7. Nukleinsäure nach einem der Ansprüche 1 bis 6, die geeignet ist, eine Reaktion zur Nukleinsäuresynthese zu initiieren, um eine Nukleinsäure mit einer dem Hepatitis C Virus entsprechenden Nukleotidsequenz zu bilden.

8. Nukleinsäure nach einem der Ansprüche 1 bis 6, die eine Sonde zum Nachweis eines Hybridisierungsprodukts aufweist.

9. Nukleinsäure nach einem der Ansprüche 1 bis 6, die ein Mittel zum Erleichtern des Abtrennens eines Hybridisierungsprodukts von der Lösung aufweist.

10. Nukleinsäure nach einem der Ansprüche 1 bis 6, die geeignet ist, die Transkription oder Translation einer viralen Nukleinsäure zu verhindern.

11. Verfahren zur Bildung eines Hybridisierungsprodukts mit einer Hepatitis C Virus Nukleinsäure, das die folgenden Schritte umfasst:
(a) Unterwerfen einer nicht natürlich vorkommenden Nukleinsäure nach einem der Ansprüche 1 bis 9, unter Bedingungen, unter denen man Hybridisierungsbedingungen schaffen kann, wobei die genannte Nukleinsäure unter Hybridisierungsbedingungen ein Hybridisierungsprodukt mit der genannten Hepatitis C Virus Nukleinsäure bilden kann; und
(b) Schaffen von Hybridisierungsbedingungen, um ein Hybridisierungsprodukt in Anwesenheit einer Hepatitis C Virus Nukleinsäure zu erzeugen.

12. Verfahren zum Nachweis eines oder mehrerer Hepatitis C Virus Genotypen, das folgende Schritte umfasst:
(a) Unterwerfen einer nicht natürlich vorkommenden Nukleinsäure nach einem der Ansprüche 1 bis 9, unter Bedingungen, unter denen man Hybridisierungsbedingungen schaffen kann,
(b) Schaffen von Hybridisierungsbedingungen, um ein Hybridisierungsprodukt in Anwesenheit einer Hepatitis C Virus Nukleinsäure zu erzeugen; und
(c) Kontrollieren der Bildung eines Hybridisierungsprodukts mit der nicht natürlich vorkommenden Nukleinsäure, wobei das Hybridisierungsprodukt auf die Anwesenheit des Hepatitis C Virus Genotyps hinweist.

## Patentansprüche (Patentansprüche für folgende(n) Vertragsstaat(en): GR, ES)

1. Verfahren zur Bildung eines Hybridisierungsprodukts mit einer Hepatitis C Virus Nukleinsäure, das die folgenden Schritte umfasst:
(a) Aussetzen einer nicht natürlich vorkommenden Nukleinsäure, die eine nicht-HCV-1 Nukleotidsequenz umfasst, die aus 8 bis 252 aufeinanderfolgenden Nukleotiden besteht, die vollständig homolog oder vollständig komplementär zu einer Nukleotidsequenz aus der 5'UT-Region eines Hepatitis C Virus sind, und aus einer Sequenz innerhalb der SEQ ID Nr. 34 bis 49 ausgewählt ist, und wobei die genannte Sequenz weder HCV-J1 noch HCV-J4 ist, unter Bedingungen, unter denen Hybridisierungsbedingungen auferlegt werden können, wobei die genannte Nukleinsäure geeignet ist, ein Hybridisierungsprodukt mit der genannten Hepatitis C Virus Nukleinsäure unter Hybridisierungsbedingungen zu bilden; und
(b) Anwenden von Hybridisierungsbedingungen, um ein Hybridisierungsprodukt in Anwesenheit einer Hepatitis C Virus Nukleinsäure zu bilden.

2. Verfahren zur Bildung eines Hybridisierungsprodukts mit einer Hepatitis C Virus Nukleinsäure, das die folgenden Schritte umfasst:
(a) Aussetzen einer nicht natürlich vorkommenden Nukleinsäure, die eine nicht-HCV-1 Nukleotidsequenz umfasst, die aus 8 bis 180 aufeinanderfolgenden Nukleotiden besteht, die vollständig homolog oder vollständig komplementär zu einer Nukleotidsequenz aus der 5'UT-Region eines Hepatitis C Virus sind, und aus einer Sequenz innerhalb der SEQ ID Nr. 50 und 51 ausgewählt ist, und wobei die genannte Sequenz weder HCV-J1 noch HCV-J4 ist, unter Bedingungen, unter denen Hybridisierungsbedingungen auferlegt werden können, wobei die genannte Nukleinsäure geeignet ist, ein Hybridisierungsprodukt mit der genannten Hepatitis C Virus Nukleinsäure unter Hybridisierungsbedingungen zu bilden; und
(b) Anwenden von Hybridisierungsbedingungen, um ein Hybridisierungsprodukt in Anwesenheit einer Hepatitis C Virus Nukleinsäure zu bilden.

3. Verfahren nach Anspruch 1, wobei die Nukleinsäure einer Nukleotidsequenz innerhalb der 5'UT-Region eines ersten Hepatitis C Virengenotyps entspricht und wobei die genannte nicht-HCV-1 Nukleotidsequenz aus einer Sequenz innerhalb der SEQ ID Nr. 34 bis 38 ausgewählt ist.

4. Verfahren nach Anspruch 1, wobei sich die Nukleinsäure innerhalb der 5'UT-Region eines zweiten Hepatitis C Virengenotyps befindet und wobei die genannte nicht-HCV-1 Nukleotidsequenz aus einer Sequenz innerhalb der SEQ ID Nr. 39 bis 45 ausgewählt ist.

5. Verfahren nach Anspruch 1, wobei sich die Nukleinsäure innerhalb der 5'UT-Region eines dritten Hepatitis C Virengenotyps befindet und wobei die genannte nicht-HCV-1 Nukleotidsequenz aus einer Sequenz innerhalb der SEQ ID Nr. 46 und 47 ausgewählt ist.

6. Verfahren nach Anspruch 1, wobei sich die Nukleinsäure innerhalb der 5'UT-Region eines vierten Hepatitis C Virengenotyps befindet und wobei die genannte nicht-HCV-1 Nukleotidsequenz aus einer Sequenz innerhalb der SEQ ID Nr. 48 und 49 ausgewählt ist.

7. Verfahren nach einem der Ansprüche 1 bis 6, wobei die Nukleinsäure geeignet ist, eine Reaktion zur Nukleinsäuresynthese zu initiieren, um eine Nukleinsäure mit einer dem Hepatitis C Virus entsprechenden Nukleotidsequenz zu bilden.

8. Verfahren nach einem der Ansprüche 1 bis 6, wobei die Nukleinsäure eine Sonde zum Nachweis eines Hybridisierungsprodukts aufweist.

9. Verfahren nach einem der Ansprüche 1 bis 6, wobei die Nukleinsäure ein Unterstützungsmittel zum Trennen eines Hybridisierungsprodukts aus der Lösung aufweist.

10. Verfahren zum Nachweisen eines oder mehrerer Hepatitis C Virengenotypen, das folgende Schritte umfasst:
(a) Aussetzen einer nicht natürlich vorkommenden Nukleinsäure, die eine nicht-HCV-1 Nukleotidsequenz umfasst, die aus 8 bis 252 aufeinanderfolgenden Nukleotiden besteht, die vollständig homolog oder vollständig komplementär zu einer Nukleotidsequenz aus der 5'UT-Region eines Hepacitis C Virus sind, und aus einer Sequenz innerhalb der SEQ ID Nr. 34 bis 49 ausgewählt ist, und wobei die genannte Sequenz weder HCV-J1 noch HCV-J4 ist, unter Bedingungen, unter denen Hybridisierungsbedingungen auferlegt werden können,
(b) Anwenden von Hybridisierungsbedingungen, um ein Hybridisierungsprodukt in Anwesenheit einer Hepatitis C Virus Nukleinsäure zu bilden; und
(c) Überwachen der nicht natürlich vorkommenden Nukleinsäure in Hinsicht auf die Bildung eines Hybridisierungsprodukts, wobei das Hybridisierungsprodukt auf die Anwesenheit des Hepatitis C Virengenotyps hinweisend ist.

11. Verfahren zum Nachweisen eines oder mehrerer Hepatitis C Virengenotypen, das folgende Schritte umfasst:
(a) Aussetzen einer nicht natürlich vorkommenden Nukleinsäure, die eine nicht-HCV-1 Nukleotidsequenz umfasst, die aus 8 bis 180 aufeinanderfolgenden Nukleotiden besteht, die vollständig homolog oder vollständig komplementär zu einer Nukleotidsequenz aus der 5'UT-Region eines Hepatitis C Virus ist und aus einer Sequenz innerhalb der SEQ ID Nr. 50 und 51 ausgewählt ist, und wobei die genannte Sequenz weder HCV-J1 noch HCV-J4 ist, unter Bedingungen, unter denen Hybridisierungsbedingungen auferlegt werden können,
(b) Anwenden von Hybridisierungsbedingungen, um ein Hybridisierungsprodukt in Anwesenheit einer Hepatitis C Virus Nukleinsäure zu bilden; und
(c) Überwachen der nicht natürlich vorkommenden Nukleinsäure in Hinsicht auf die Bildung eines Hybridisisrungsprodukts, wobei das Hybridisierungsprodukt auf die Anwesenheit des Hepatitis C Virengenotyps hinweisend ist.

12. Verfahren nach Anspruch 10, wobei die Nukleinsäure einer Nukleotidsequenz innerhalb der 5'UT-Region eines ersten Hepatitis C Virengenotyps entspricht und wobei die genannte nicht-HCV-1 Nukleotidsequenz aus einer Sequenz innerhalb der SEQ ID Nr. 34 bis 38 ausgewählt ist.

13. Verfahren nach Anspruch 10, wobei sich die Nukleinsäure innerhalb der 5'UT-Region eines zweiten Hepatitis C Virengenotyps befindet und wobei die genannte nicht-HCV-1 Nukleotidsequenz aus einer Sequenz innerhalb der SEQ ID Nr. 39 bis 45 ausgewählt ist.

14. Verfahren nach Anspruch 10, wobei sich die Nukleinsäure innerhalb der 5'UT-Region eines dritten Hepatitis C Virengenotyps befindet und wobei die genannte nicht-HCV-1 Nukleotidsequenz aus einer Sequenz innerhalb der SEQ ID Nr. 46 und 47 ausgewählt ist.

15. Verfahren nach Anspruch 10, wobei sich die Nukleinsäure innerhalb der 5'UT-Region eines vierten Hepatitis C Virengenotyps befindet und wobei die genannte nicht-HCV-1 Nukleotidsequenz aus einer Sequenz innerhalb der SEQ ID Nr. 48 und 49 ausgewählt ist.

16. Verfahren nach einem der Ansprüche 10 bis 15, wobei die Nukleinsäure geeignet ist, eine Reaktion zur Nukleinsäuresynthese zu initiieren, um eine Nukleinsäure mit einer dem Hepatitis C Virus entsprechenden Nukleotidsequenz zu bilden.

17. Verfahren nach einem der Ansprüche 10 bis 15, wobei die Nukleinsäure eine Sonde zum Nachweis eines Hybridisierungsprodukts auf weist.

18. Verfahren nach einem der Ansprüche 10 bis 15, wobei die Nukleinsäure ein Unterstützungsmittel zum Trennen eines Hybridisierungsprodukts aus der Lösung aufweist.

## Revendications (Revendications pour l'(les) Etat(s) contractant(s) suivant(s): AT, BE, CH, LI, DE, DK, FR, GB, IT, LU, MC, NL, SE)

1. Acide nucléique n'existant pas à l'état naturel comprenant une séquence nucléotidique non-HCV-1 consistant en 8 à 252 nucléotides contigus qui sont pleinement homologues à ou pleinement complémentaires d'une séquence nucléotidique de la région 5'UT d'un virus de l'hépatite C et choisis parmi une des séquences SEQ lD n° 34 à 49 et dans lequel ladite séquence n'est pas HCV-J1 ou HCV-J4.

2. Acide nucléique n'existant pas à l'état naturel comprenant une séquence nucléotididique non-HCV-1 consistant en 8 à 180 nucléotides contigus qui sont pleinement homologues à ou pleinement complémentaires d'une séquence nucléotidique de la région 5'UT d'un virus de l'hépatite C et choisi parmi une des séquences SEQ lD n° 50 et 51 et dans lequel ladite séquence n'est pas HCV-J1 ou HCV-J4.

3. Acide nucléique selon la revendication 1, correspondant à une séquence nucléotidique à l'intérieur de la région 5'UT d'un premier génotype de virus de l'hépatite C dans lequel ladite séquence nucléotidique non-HCV-1 est choisie parmi une des séquences SEQ ID N° 34 à 38.

4. Acide nucléique selon la revendication 1, à l'intérieur de la région 5'UT d'un second génotype de virus de l'hépatite C dans lequel ladite séquence nucléotidique non-HCV-1 est choisie parmi une des séquences SEQ ID N° 39 à 45.

5. Acide nucléique selon la revendication 1, à l'intérieur de la région 5'UT d'un troisième génotype de virus de l'hépatite C dans lequel ladite séquence nucléotidique non-HCV-1 est choisie parmi une des séquences SEQ ID N° 46 et 47.

6. Acide nucléique selon la revendication 1, à l'intérieur de la région 5'UT d'un quatrième génotype de virus de l'hépatite C dans lequel ladite séquence nucléotidique non-HCV-1 est choisie parmi une des séquences SEQ lD N° 48 et 49.

7. Acide nucléique selon l'une quelconque des revendications 1 à 6 qui est capable d'amorcer une réaction pour la synthèse d'un acide nucléique pour former un acide nucléique possédant une séquence nucléotidique correspondant au virus de l'hépatite C.

8. Acide nucléique selon l'une quelconque des revendications 1 à 6, possédant un moyen de marquage pour la détection d'un produit d'hybridation.

9. Acide nucléique selon l'une quelconque des revendications 1 à 6, possédant des moyens de support pour la séparation d'un produit d'hybridation à partir d'une solution.

10. Acide nucléique selon l'une quelconque des revendications 1 à 6 qui est capable de prévenir la transcription ou la traduction d'un acide nucléique viral.

11. Procédé de formation d'un produit d'hybridation avec un acide nucléique de virus de l'hépatite C comprenant les étapes suivantes
(a) placer un acide nucléique n'existant pas à l'état naturel selon l'une quelconque des revendications 1 à 9 dans des conditions dans lesquelles les conditions d'hybridation peuvent être imposées, ledit acide nucléique étant capable de former un produit d'hybridation avec ledit acide nucléique de virus de l'hépatite C dans des conditions d'hybridation ; et
(b) imposer des conditions d'hybridation pour former un produit d'hybridation en présence d'acide nucléique de virus de l'hépatite C.

12. Procédé de détection d'un ou plusieurs génotypes de virus de l'hépatite C comprenant les étapes suivantes :
(a) placer un acide nucléique n'existant pas à l'état naturel selon l'une quelconque des revendications 1 à 9 dans des conditions dans lesquelles les conditions d'hybridation peuvent être imposées ;
(b) imposer des conditions d'hybridation pour former un produit d'hybridation en présence d'un acide nucléique de virus de l'hépatite C ; et
(c) contrôler l'acide nucléique n'existant pas à l'état naturel pour la formation d'un produit d'hybridation, lequel produit d'hybridation indique la présence du génotype de virus de l'hépatite C.

## Revendications (Revendications pour l'(les) Etat(s) contractant(s) suivant(s): GR, ES)

1. Procédé de formation d'un produit d'hybridation avec un acide nucléique de virus de l'hépatite C comprenant les étapes suivantes:
(a) placer un acide nucléique n'existant pas à l'état naturel comprenant une séquence nucléotidique non-HCV-1 consistant en 8 à 252 nucléotides contigus qui sont pleinement homologues à ou pleinement complémentaires d'une séquence nucléotidique de la région 5' UT d'un virus de l'hépatite C et choisis parmi une des séquences SEQ ID n° 34 à 49 et dans lequel ladite séquence n'est pas HCV-J1 ou HCV-J4 dans des conditions dans lesquelles les conditions d'hybridation peuvent être imposées, ledit acide nucléique étant capable de former un produit d'hybridation avec ledit acide nucléique de virus de l'hépatite C dans des conditions d'hybridation; et
(b) imposer des conditions d'hybridation pour former un produit d'hybridation en présence d'acide nucléique de virus de l'hépatite C.

2. Procédé de formation d'un produit d'hybridation avec un acide nucléique de virus de l'hépatite C comprenant les étapes suivantes:
(a) placer un acide nucléique n'existant pas à l'état naturel comprenant une séquence nucléotidique non-HCV-1 consistant en 8 à 180 nucléotides contigus qui sont pleinement homologues à ou pleinement complémentaires d'une séquence nucléotidique de la région 5' UT d'un virus de l'hépatite C et choisis parmi une des séquences SEQ ID n° 50 à 51 et dans lequel ladite séquence n'est pas HCV-J1 ou HCV-J4 dans des conditions dans lesquelles les conditions d'hybridation peuvent être imposées, ledit acide nucléique étant capable de former un produit d'hybridation avec ledit acide nucléique de virus de l'hépatite C dans des conditions d'hybridation; et
(b) imposer des conditions d'hybridation pour former un produit d'hybridation en présence d'acide nucléique de virus de l'hépatite C.

3. Procédé selon la revendication 1, dans lequel ledit acide nucléique correspondant à une séquence nucléotidique à l'intérieur de la région 5'UT d'un premier génotype de virus de l'hépatite C et dans lequel ladite séquence nucléotidique non-HCV-1 est choisie parmi une des séquences SEQ ID N° 34 à 38.

4. Procédé selon la revendication 1, dans lequel ledit acide nucléique se prouvant à l'intérieur de la région 5'UT d'un second génotype de virus de l'hépatite C et dans lequel ladite séquence nucléotidique non-HCV-1 est choisie parmi une des séquences SEQ ID N° 39 à 45.

5. Procédé selon la revendication 1, dans lequel ledit acide nucléique se trouvant à l'intérieur de la région 5'UT d'un troisième génocype de virus de l'hépatite C et dans lequel ladite séquence nucléotidique non-HCV-1 est choisie parmi une des séquences SEQ ID N° 46 et 47.

6. Procédé selon la revendication 1, dans lequel ledit acide nucléique se trouvant à l'intérieur de la région 5'UT d'un quatrième génotype de virus de l'hépatite C dans lequel ladite séquence nucléotidique non-HCV-1 est choisie parmi une des séquences SEQ ID N° 48 et 49.

7. Procédé selon l'une quelconque des revendications 1 à 6, dans lequel ledit acide nucléique est capable d'amorcer une réaction pour la synthèse d'un acide nucléique pour former un acide nucléique possédant une séquence nucléotidique correspondant au virus de l'hépatite C.

8. Procédé selon l'une quelconque des revendications 1 à 6, dans lequel ledit acide nucléique possède un moyen de marquage pour la détection d'un produit d'hybridation.

9. Procédé selon l'une quelconque des revendications 1 à 6, dans lequel ledit acide nucléique possède des moyens de support pour la séparation d'un produit d'hybridation à partir d'une solution.

10. Procédé de détection d'un ou plusieurs génotype de virus de l'hépatite C comprenant les étapes suivantes:
(a) placer un acide nucléique n'existant pas à l'état naturel comprenant une séquence nucléotidique non-HCV-1 consistant en 8 à 252 nucléotides contigus qui sont pleinement homologues à ou pleinement complémentaires d'une séquence nucléotidique de la région 5'UT d'un virus de l'hépatite C et choisis parmi une des séquences SEQ ID n° 34 à 49 et dans lequel ladite séquence n'est pas HCV-J1 ou HCV-J4 dans des conditions dans lesquelles les conditions d'hybridation peuvent être imposées;
(b) imposer des conditions d'hybridation pour former un produit d'hybridation en présence d'un acide nucléique de virus de l'hépatite C; et
(c) contrôler l'acide nucléique n'existant pas à l'état naturel pour la formation d'un produit d'hybridation, lequel produit d'hybridation indique la présence du génotype de virus de l'hépatite C.

11. Procédé de détection d'un ou plusieurs génotype de virus de l'hépatite C comprenant les étapes suivantes:
(a) placer un acide nucléique n'existant pas à l'état naturel comprenant une séquence nucléotidique non-HCV-1 consistant en 8 à 180 nucléotides contigus qui sont pleinement homologues à ou pleinement complémentaires d'une séquence nucléotidique de la région 5'UT d'un virus de l'hépatite C et choisis parmi une des séquences SEQ ID n° 50 à 51 et dans lequel ladite séquence n'est pas HCV-J1 ou HCV-J4 dans des conditions dans lesquelles les conditions d'hybridation peuvent être imposées;
(b) imposer des conditions d'hybridation pour former un produit d'hybridation en présence d'un acide nucléique de virus de l'hépatite C; et
(c) contrôler l'acide nucléique n'existant pas à l'état naturel pour la formation d'un produit d'hybridation, lequel produit d'hybridation indique la présence du génotype de virus de l'hépatite C.

12. Procédé selon la revendication 10, dans lequel ledit acide nucléique correspondant à une séquence nucléotidique à l'intérieur de la région 5' UT d'un premier génotype de virus de l'hépatite C et dans lequel ladite séquence nucléotidique non-HCV-1 est choisie parmi une des séquences SEQ ID N° 34 à 38.

13. Procédé selon la revendication 10, dans lequel ledit acide nucléique se trouvant à l'intérieur de la région 5'UT d'un second génotype de virus de l'hépatite C et dans lequel ladite séquence nucléotidique non-HCV-1 est choisie parmi une des séquences SEQ ID N° 39 à 45.

14. Procédé selon la revendication 10, dans lequel ledit acide nucléique se trouvant à l'intérieur de la région 5'UT d'un troisième génotype de virus de l'hépatite C et dans lequel ladite séquence nucléotidique non-HCV-1 est choisie parmi une des séquences SEQ ID N° 46 et 47.

15. Procédé selon la revendication 10, dans lequel ledit acide nucléique se trouvant à l'intérieur de la région 5'UT d'un quatrième génotype de virus de l'hépatite C dans lequel ladite séquence nucléotidique non-HCV-1 est choisie parmi une des séquences SEQ ID N° 48 et 49.

16. Procédé selon l'une quelconque des revendications 10 à 15, dans lequel ledit acide nucléique est capable d'amorcer une réaction pour la synthèse d'un acide nucléique pour former un acide nucléique possédant une séquence nucléotidique correspondant au virus de l'hépatite C.

17. Procédé selon l'une quelconque des revendications 10 à 15, dans lequel ledit acide nucléique possède un moyen de marquage pour la détection d'un produit d'hybridation.

18. Procédé selon l'une quelconque des revendications 10 à 15, dans lequel ledit acide nucléique possède des moyens de support pour la séparation d'un produit d'hybridation à partir d'une solution.


### SEQUENCE LISTING

(1) GENERAL INFORMATION:
   (i) APPLICANT: Tai-An Cha
   (ii) TITLE OF INVENTION: HCV GENOMIC SEQUENCES FOR DIAGNOSTICS AND THERAPEUTICS
   (iii) NUMBER OF SEQUENCES: 147
   (iv) CORRESPONDENCE ADDRESS:
      (A) ADDRESSEE: Wolf, Greenfield & Sacks, P.C.
      (B) STREET: 600 Atlantic Avenue
      (C) CITY: Boston
      (D) STATE: Massachusetts
      (E) COUNTRY: USA
      (F) ZIP: 02210
   (v) COMPUTER READABLE FORM:
      (A) MEDIUM TYPE: Diskette, 5.25 inch
      (B) COMPUTER: IBM compatible
      (C) OPERATING SYSTEM: MS-DOS Version 3.3
      (D) SOFTWARE: WordPerfect 5.1
   (vi) CURRENT APPLICATION DATA:
      (A) APPLICATION NUMBER: Not Available
      (B) FILING DATE: Not Available
      (C) CLASSIFICATION: Not Available
   (vii) PRIOR APPLICATION DATA:
      (A) APPLICATION NUMBER: 07/697,326
      (B) FILING DATE: 8 May 1991
   (viii) ATTORNEY/AGENT INFORMATION:
      (A) NAME: Janiuk, Anthony J.
      (B) REGISTRATION NUMBER: 29,809
      (C) REFERENCE/DOCKET NUMBER: C0772/7000
   (ix) TELECOMMUNICATION INFORMATION:
      (A) TELEPHONE: (617) 720-3500
      (B) TELEFAX: (617) 720-2441
      (C) TELEX: EZEKIEL
(2) INFORMATION FOR SEQ ID NO: 1:
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE: (ATCC # 40394)
      (C) INDIVIDUAL ISOLATE: ns5hcvl
   (xi) SEQUENCE DESCRIPTION: SEQ ID NO; 1
   CTCCACAGTC ACTGAGAGCG ACATCCGTAC GGAGGAGGCA    40
   ATCTACCAAT GTTGTGACCT CGACCCCCAA GCCCGCGTGG    80
   CCATCAAGTC CCTCACCGAG AGGCTTTATG TTGGGGGCCC   120
   TCTTACCAAT TCAAGGGGGG AGAACTGCGG CTATCGCAGG   160
   TGCCGCGCGA GCGGCGTACT GACAACTAGC TGTGGTAACA   200
   CCCTCACTTG CTACATCAAG GCCCGGGCAG CCTGTCGAGC   240
   CGCAGGGCTC CAGGACTGCA CCATGCTCGT GTGTGGCGAC   280
   GACTTAGTCG TTATCTGTGA AAGCGCGGGG GTCCAGGAGG   320
   ACGCGGCGAG CCTGAGAGCC                         340
(2) INFORMATION FOR SEQ ID NO: 2:
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5i21
   (xi) SEQUENCE DESCRIPTION: SEQ ID NO: 2
   CTCCACAGTC ACTGAGAGCG ACATCCGTAC GGAGGAGGCA    40
   ATTTACCAAT GTTGTGACCT GGACCCCCAA GCCCGCATGG    80
   CCATCAAGTC CCTCACTGAG AGGCTTTATG TCGGGGGCCC   120
   TCTTACCAAT TCAAGGGGGG AGAACTGCGG CTACCGCAGG   160
   TGCCGCGCGA GCGGCGTACT GACAACTAGC TGTGGTAACA   200
   CCCTCACTTG CTACATCAAG GCCCGGGCAG CCTGTCGAGC   240
   CGCAGGGCTC CAGGACTGCA CCATGCTTGT GTGTGGCGAC   280
   GACTTAGTCG TTATCTGTGA AAGTGCGGGG GTCCAGGAGG   320
   ACGCGGCGAG CCTGAGAGCC                         340
(2) INFORMATION FOR SEQ ID NO: 3:
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) individual isolate: ns5pt1
   (xi) SEQUENCE DESCRIPTION: SEQ ID NO: 3
   CTCCACAGTC ACTGAGAGCG ACATCCGTAC GGAGGAGGCA     40
   ATCTACCAAT GTTGTGATCT GGACCCCCAA GCCCGCGTGG     80
   CCATCAAGTC CCTCACTGAG AGGCTTTACG TTGGGGGCCC    120
   TCTTACCAAT TCAAGGGGGG AGAACTGCGG CTACCGCAGG    160
   TGCCGGGCGA GCGGCGTACT GACAACTAGC TGTGGTAATA    200
   CCCTCACTTG CTACATCAAG GCCCGGGCAG CCTGTCGAGC    240
   CGCAGGGCTC CGGGACTGCA CCATGCTCGT GTGTGGTGAC    280
   GACTTGGTCG TTATCTGTGA GAGTGCGGGG GTCCAGGAGG    320
   ACGCGGCGAG CCTGAGAGCC                          340
(2) INFORMATION FOR SEQ ID NO: 4
   (i) SEQUENCE CHARACTERISTICS;
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5gm2
   (xi) SEQUENCE DESCRIPTION: SEQ ID NO: 4
   CTCTACAGTC ACTGAGAACG ACATCCGTAC GGAGGAGGCA     40
   ATTTACCAAT GTTGTGACCT GGACCCCCAA GCCCGCGTGG     80
   CCATCAAGTC CCTCACTGAG AGGCTTTATG TTGGGGGCCC   120
   CCTTACCAAT TCAAGGGGGG AAAACTGCGG CTATCGCAGG   160
   TGCCGCGCGA GCGGCGTACT GACAACTAGC TGTGGTAACA   200
   CCCTCACTTG CTACATTAAG GCCCGGGCAG CCTGTCGAGC   240
   CGCAGGGCTC CAGGACTGCA CCATGCTCGT GTGTGGCGAC   280
   GACTTAGTCG TTATCTGTGA GAGTGCGGGA GTCCAGGAGG   320
   ACGCGGCGAA CTTGAGAGCC                         340
(2) INFORMATION FOR SEQ ID NO: 5
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5us17
   (Xi) SEQUENCE DESCRIPTION: SEQ ID NO: 5
   CTCCACAGTC ACTGAGAGCG ATATCCGTAC GGAGGAGGCA    40
   ATCTACCAGT GTTGTGACCT GGACCCCCAA GCCCGCGTGG    80
   CCATCAAGTC CCTCACCGAG AGGCTTTATG TCGGGGGCCC   120
   TCTTACCAAT TCAAGGGGGG AAAACTGCGG CTATCGCAGG   160
   TGCCGCGCAA GCGGCGTACT GACAACTAGC TGTGGTAACA   200
   CCCTCACTTG TTACATCAAG GCCCAAGCAG CCTGTCGAGC   240
   CGCAGGGCTC CGGGACTGCA CCATGCTCGT GTGTGGCGAC   280
   GACTTAGTCG TTATCTGTGA AAGTCAGGGA GTCCAGGAGG   320
   ATGCAGCGAA CCTGAGAGCC                         340
(2) INFORMATION FOR SEQ ID NO: 6
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5sp2
   (xi) SEQUENCE DESCRIPTION: SEQ ID NO: 6
   CTCTACAGTC ACTGAGAGCG ATATCCGTAC GGAGGAGGCA    40
   ATCTACCAAT GTTGTGACCT GGACCCCGAA GCCCGTGTGG    80
   CCATCAAGTC CCTCACTGAG AGGCTTTATG TTGGGGGCCC   120
   TCTTACCAAT TCAAGGGGGG AGAACTGCGG CTACCGCAGG   160
   TGCCGCGCAA GCGGCGTACT GACGACTAGC TGTGGTAATA   200
   CCCTCACTTG TTACATCAAG GCCCGGGCAG CCTGTCGAGC   240
   CGCAGGGCTC CAGGACTGCA CCATGCTCGT GTGTGGCGAC   280
   GACCTAGTCG TTATCTGCGA AAGTGCGGGG GTCCAGGAGG   320
   ACGCGGCGAG CCTGAGAGCC                         340
(2) INFORMATION FOR SEQ ID NO: 7
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5j1
   (xi) SEQUENCE DESCRIPTION: SEQ ID NO: 7
   CTCCACAGTC ACTGAGAATG ACACCCGTGT TGAGGAGTCA    40
   ATTTACCAAT GTTGTGACTT GGCCCCCGAA GCCAGACAGG    80
   CCATAAGGTC GCTCACAGAG CGGCTCTATG TCGGGGGTCC   120
   TATGACTAAC TCCAAAGGGC AGAACTGCGG CTATCGCCGG   160
   TGCCGCGCGA GCGGCGTGCT GACGACTAGC TGCGGTAATA   200
   CCCTCACATG CTACCTGAAG GCCACAGCGG CCTGTCGAGC   240
   TGCCAAGCTC CAGGACTGCA CGATGCTCGT GAACGGAGAC   280
   GACCTTGTCG TTATCTGTGA AAGCGCGGGG AACCAAGAGG   320
   ACGCGGCAAG CCTACGAGCC                         340
(2) INFORMATION FOR SEQ ID NO: 8
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5k1
   (xi) SEQUENCE DESCRIPTION: SEQ ID NO: 8
   CTCAACGGTC ACTGAGAATG ACATCCGTGT TGAGGAGTCA    40
   ATTTACCAAA GTTGTGACTT GGCCCCCGAG GCCAGACAAG    80
   CCATAAGGTC GCTCACAGAG CGGCTTTACA TCGGGGGCCC   120
   CCTGACTAAT TCAAAAGGGC AGAACTGCGG CTATCGCCGA   160
   TGCCGCGCCA GCGGTGTGCT GACGACTAGC TGCGGTAATA   200
   CCCTCACATG TTACTTGAAG GCCACTGCGG CCTGTAGAGC   240
   TGCGAAGCTC CAGGACTGCA CGATGCTCGT GTGCGGAGAC   280
   GACCTTGTCG TTATCTGTGA AAGCGCGGGA ACCCAGGAGG   320
   ATGCGGCGAG CCTACGAGTC                         340
(2) INFORMATION FOR SEQ ID NO: 9
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5k1.1
   (xi) SEQUENCE DESCRIPTION: SEQ ID NO: 9
   CTCAACGGTC ACCGAGAATG ACATCCGTGT TGAGGAGTCA    40
   ATTTATCAAT GTTGTGCCTT GGCCCCCGAG GCTAGACAGG    80
   CCATAAGGTC GCTCACAGAG CGGCTTTATA TCGGGGGCCC   120
   CCTGACCAAT TCAAAGGGGC AGAACTGCGG TTATCGCCGG   160
   TGCCGCGCCA GCGGCGTACT GACGACCAGC TGCGGTAATA   200
   CCCTTACATG TTACTTGAAG GCCTCTGCAG CCTGTCGAGC   240
   CGCGAAGCTC CAGGACTGCA CGATGCTCGT GTGTGGGGAC   280
   GACCTTGTCG TTATCTGTGA AAGCGCGGGA ACCCAGGAGG   320
   ACGCGGCGAA CCTACGAGTC                         340
(2) INFORMATION FOR SEQ ID NO: 10
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5gh6
   (xi) SEQUENCE DESCRIPTION: SEQ ID NO: 10
   CTCAACGGTC ACTGAGAGTG ACATCCGTGT CGAGGAGTCG    40
   ATTTACCAAT GTTGTGACTT GGCCCCCGAA GCCAGGCAGG    80
   CCATAAGGTC GCTCACCGAG CGACTTTATA TCGGGGGCCC   120
   CCTGACTAAT TCAAAAGGGC AGAACTGCGG TTATCGCCGG   160
   TGCCGCGCGA GCGGCGTGCT GACGACTAGC TGCGGTAATA   200
   CCCTCACATG TTACTTGAAG GCCTCTGCAG CCTGTCGAGC   240
   TGCAAAGCTC CAGGACTGCA CGATGCTCGT GAACGGGGAC   280
   GACCTTGTCG TTATCTGCGA GAGCGCGGGA ACCCAAGAGG   320
   ACGCGGCGAG CCTACGAGTC                         340
(2) INFORMATION FOR SEQ ID NO: 11
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5sp1
   (xi) SEQUENCE DESCRIPTION: SEQ ID NO: 11
   CTCCACAGTC ACTGAGAGTG ACATCCGTGT TGAGGAGTCA    40
   ATTTACCAAT GTTGTGACTT GGCCCCCGAA GCCAGACAGG    80
   CTATAAGGTC GCTCACAGAG CGGCTGTACA TCGGGGGTCC   120
   CCTGACTAAT TCAAAAGGGC AGAACTGCGG CTATCGCCGG   160
   TGCCGCGCAA GCGGCGTGCT GACGACTAGC TGCGGTAACA   200
   CCCTCACATG TTACTTGAAG GCCTCTGCGG CCTGTCGAGC   240
   TGCGAAGCTC CAGGACTGCA CGATGCTCGT GTGCGGTGAC   280
   GACCTTGTCG TTATCTGTGA GAGCGCGGGA ACCCAAGAGG   320
   ACGCGGCGAG CCTACGAGTC                         340
(2) INFORMATION FOR SEQ ID NO: 12
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) individual isolate: ns5sp3
   (xi) SEQUENCE DESCRIPTION: SEQ ID NO; 12
   CTCAACAGTC ACTGAGAGTG ACATCCGTGT TGAGGAGTCA     40
   ATCTACCAAT GTTGTGACTT GGCCCCCGAA GCCAGACAGG     80
   CTATAAGGTC GCTCACAGAG CGGCTTTACA TCGGGGGTCC    120
   CCTGACTAAT TCAAAAGGGC AGAACTGCGG CTATCGCCGG    160
   TGCCGCGCAA GCGGCGTGCT GACGACTAGC TGCGGTAATA    200
   CCCTCACATG TTACCTGAAG GCCAGTGCGG CCTGTCGAGC    240
   TGCGAAGCTC CAGGACTGCA CAATGCTCGT GTGCGGTGAC    280
   GACCTTGTCG TTATCTGTGA GAGCGCGGGG ACCCAAGAGG    320
   ACGCGGCGAG CCTACGAGTC                          340
(2) INFORMATION FOR SEQ ID NO: 13
   (i) _{.} SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5k2
   (xi) SEQUENCE DESCRIPTION: SEQ ID NO: 13
   CTCAACCGTC ACTGAGAGAG ACATCAGAAC TGAGGAGTCC     40
   ATATACCGAG CCTGCTCCCT GCCTGAGGAG GCTCACATTG     80
   CCATACACTC GCTGACTGAG AGGCTCTACG TGGGAGGGCC    120
   CATGTTCAAC AGCAAGGGCC AGACCTGCGG GTACAGGCGT    160
   TGCCGCGCCA GCGGGGTGCT CACCACTAGC ATGGGGAACA    200
   CCATCACATG CTATGTAAAA GCCCTAGCGG CTTGCAAGGC    240
   TGCAGGGATA GTTGCACCCT CAATGCTGGT ATGCGGCGAC    280
   GACTTAGTTG TCATCTCAGA AAGCCAGGGG ACTGAGGAGG    320
   ACGAGCGGAA CCTGAGAGCT                          340
(2) INFORMATION FOR SEQ ID NO: 14
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5arg8
   (xi) SEQUENCE DESCRIPTION: SEQ ID NO: 14
   CTCTACAGTC ACGTAAAAGG ACATCACATC CTAGGAGTCC    40
   ATCTACCAGT CCTGTTCACT GCCCGAGGAG GCTCGAACTG    80
   CTATACACTC ACTGACTGAG AGACTATACG TAGGGGGGCC   120
   CATGACAAAC AGCAAGGGCC AATCCTGCGG GTACAGGCGT   160
   TGCCGCGCGA GCGCAGTGCT CACCACCAGC ATGGGCAACA   200
   CACTCACGTG CTACGTAAAA GCCAGGGCGG CGTGTAACGC   240
   CGCGGGGATT GTTGCTCCCA CCATGCTGGT GTGCGGTGAC   280
   GACCTGGTCG TCATCTCAGA GAGTCAAGGG GCTGAGGAGG   320
   ACGAGCAGAA CCTGAGAGTC                         340
(2) INFORMATION FOR SEQ ID NO: 15
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5i10
   (xi) SEQUENCE DESCRIPTION: SEQ ID NO: 15
   CTCTACAGTC ACAGAGAGGG ACATCAGAAC CGAGGAGTCC    40
   ATCTATCTGT CCTGCTCACT GCCTGAGGAG GCCCGAACTG    80
   CTATACACTC ACTGACTGAG AGACTGTACG TAGGGGGGCC   120
   CATGACAAAC AGCAAGGGGC AATCCTGCGG GTACAGGCGT   160
   TGCCGCGCGA GCGGAGTGCT CACCACCAGC ATGGGCAACA   200
   CGCTCACGTG CTACGTGAAA GCCAGAGCGG CGTGTAACGC   240
   CGCGGGCATT GTTGCTCCCA CCATGTTGGT GTGCGGCGAC   280
   GACCTGGTTG TCATCTCAGA GAGTCAGGGG GTCGAGGAAG   320
   ATGAGCGGAA CCTGAGAGTC                         340
(2) INFORMATION FOR SEQ ID NO: 16
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5arg6
   (xi) SEQUENCE DESCRIPTION: SEQ ID NO: 16
   CTCTACAGTC ACGGAGAGGG ACATCAGAAC CGAGGAGTCC    40
   ATCTATCTGT CCTGTTCACT GCCTGAGGAG GCTCGAACTG    80
   CCATACACTC ACTGACTGAG AGGCTGTACG TAGGGGGGCC   120
   CATGACAAAC AGCAAAGGGC AATCCTGCGG GTACAGGCGT   160
   TGCCGCGCGA GCGGAGTGCT CACCACCAGC ATGGGTAACA   200
   CACTCACGTG CTACGTGAAA GCTAAAGCGG CATGTAACGC   240
   CGCGGGCATT GTTGCCCCCA CCATGTTGGT GTGCGGCGAC   280
   GACCTAGTCG TCATCTCAGA GAGTCAAGGG GTCGAGGAGG   320
   ATGAGCGAAA CCTGAGAGCT                         340
(2) INFORMATION FOR SEQ ID NO: 17
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5k2b
   (xi) SEQUENCE DESCRIPTION: SEQ ID NO: 17
   CTCAACCGTC ACGGAGAGGG ACATAAGAAC AGAAGAATCC    40
   ATATATCAGG GTTGTTCCCT GCCTCAGGAG GCTAGAACTG    80
   CTATCCACTC GCTCACTGAG AGACTCTACG TAGGAGGGCC   120
   CATGACAAAC AGCAAGGGAC AATCCTGCGG TTACAGGCGT   160
   TGCCGCGCCA GCGGGGTCTT CACCACCAGC ATGGGGAATA   200
   CCATGACATG CTACATCAAA GCCCTTGCAG CGTGCAAAGC   240
   TGCAGGGATC GTGGACCCTA TCATGCTGGT GTGTGGAGAC   280
   GACCTGGTCG TCATCTCGGA GAGCGAAGGT AACGAGGAGG   320
   ACGAGCGAAA CCTGAGAGCT                         340
(2) INFORMATION FOR SEQ ID NO: 18
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5sa283
   (xi) SEQUENCE DESCRIPTION: SEQ ID NO: 18
(2) INFORMATION FOR SEQ ID NO: 19
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5sa156
   (xi) SEQUENCE DESCRIPTION: SEQ ID NO: 19
   CTCGACCGTT ACCGAACATG ACATAATGAC TGAAGAGTCC    40
   ATTTACCAAT CATTGTACTT GCAGCCTGAG GCACGCGCGG    80
   CAATACGGTC ACTCACCCAA CGCCTGTACT GTGGAGGCCC   120
   CATGTATAAC AGCAAGGGGC AACAATGTGG TTACCGTAGA   160
   TGCCGCGCCA GCGGCGTCTT CACCACCAGT ATGGGCAACA   200
   CCATGACGTG CTACATCAAG GCTTCAGCCG CCTGTAGAGC   240
   TGCAAAGCTC CAGGACTGCA CGCTCCTGGT GTGTGGTGTG   280
   ACCTTGGTGG CCATTTGCGA GAGCCAAGGG ACGCACGAGG   320
   ATGAAGCGTG CCTGAGAGTC                         340
(2) INFORMATION FOR SEQ ID NO: 20
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5i11
   (xi) SEQUENCE DESCRIPTION: SEQ ID NO: 20
   CTCTACTGTC ACTGAACAGG ACATCAGGGT GGAAGAGGAG    40
   ATATACCAGT GCTGTAACCT TGAACCGGAG GCCAGGAAAG    80
   TGATCTCCTC CCTCACGGAG CGGCTTTACT GCGGGGGCCC   120
   TATGTTCAAC AGCAAGGGGG CCCAGTGTGG TTATCGCCGT   160
   TGCCGTGCTA GTGGAGTCCT GCCTACCAGC TTCGGCAACA   200
   CAATCACTTG TTACATCAAG GCTAGAGCGG CTTCGAAGGC   240
   CGCAGGCCTC CGGAACCCGG ACTTTCTTGT CTGCGGAGAT   280
   GATCTGGTCG TGGTGGCTGA GAGTGATGGC GTCGACGAGG   320
   ATAGAGCAGC CCTGAGAGCC                         340
(2) INFORMATION FOR SEQ ID NO: 21
   (i) SEQUENCE CHARACTERISTICS:
      (A) LENGTH: 340 nucleotides
      (3) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5i4
   (xi) SEQUENCE DESCRIPTION: SEQ ID NO: 21
   CTCGACTGTC ACTGAACAGG ACATCAGGGT GGAAGAGGAG    40
   ATATACCAAT GCTGTAACCT TGAACCGGAG GCCAGGAAAG    80
   TGATCTCCTC CCTCACGGAG CGGCTTTACT GCGGGGGCCC   120
   TATGTTCAAT AGCAAGGGGG CCCAGTGTGG TTATCGCCGT   160
   TGCCGTGCTA GTGGAGTTCT GCCTACCAGC TTCGGCAACA   200
   CAATCACTTG TTACATCAAG GCTAGAGCGG CTGCGAAGGC   240
   CGCAGGGCTC CGGACCCCGG ACTTTCTCGT CTGCGGAGAT   280
   GATCTGGTTG TGGTGGCTGA GAGTGATGGC GTCGACGAGG   320
   ATAGAACAGC CCTGCGAGCC                         340
(2) INFORMATION FOR SEQ ID NO: 22
   (i) SEQUENCE CHARACTERISTICS:
      (A) LENGTH: 340 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ns5gh8
   (xi) SEQUENCE DESCRIPTION: SEQ ID NO: 22
   CTCAACTGTC ACTGAACAGG ACATCAGGGT GGAAGAGGAG    40
   ATATACCAAT GCTGTAACCT TGAACCGGAG GCCAGGAAAG    80
   TGATCTCCTC CCTCACGGAA CGGCTTTACT GCGGGGGCCC   120
   TATGTTCAAC AGCAAGGGGG CCCAGTGTGG TTATCGCCGT   160
   TGCCGTGCCA GTGGAGTTCT GCCTACCAGC TTCGGCAACA   200
   CAATCACTTG TTACATCAAA GCTAGAGCGG CTGCCGAAGC   240
   CGCAGGCCTC CGGAACCCGG ACTTTCTTGT CTGCGGAGAT   280
   GATCTGGTTG TGGTGGCTGA GAGTGATGGC GTCAATGAGG   320
   ATAGAGCAGC CCTGGGAGCC                         340
(2) INFORMATION FOR SEQ ID NO: 23
   (i) SEQUENCE CHARACTERISTICS:
      (A) LENGTH: 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE: (ATCC # 40394)
      (C) INDIVIDUAL ISOLATE: hcv1
   (xi)- SEQUENCE DESCRIPTION: SEQ ID NO: 23
(2) INFORMATION FOR SEQ ID NO: 24
   (i) SEQUENCE CHARACTERISTICS:
      (A) LENGTH; 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: US5
   (xi) SEQUENCE DESCRIPTION: SEQ ID NO: 24
(2) INFORMATION FOR SEQ ID NO: 25
   (i) SEQUENCE CHARACTERISTICS:
      (A) LENGTH: 100 nucleotides
      (B) TYPE: nucleic acid
      (C) STRANDEDNESS; single
      (D) TOPOLOGY: linear
   (ii) MOLECULE TYPE: DNA
   (vi) ORIGINAL SOURCE:
      (C) INDIVIDUAL ISOLATE: AUS5
   (xi) SEQUENCE DESCRIPTION: SEQ ID NO: 25
   AACGGCGCTG GTAGTAGCTC AGCTGCTCAG GGTCCCGCAA    40
   GCCATCGTGG ACATGATCGC TGGTGCCCAC TGGGGAGTCC    80
   TAGCGGGCAT AGCGTATTTT                         100
(2) INFORMATION FOR SEQ ID NO: 26
   (i) SEQUENCE CHARACTERISTICS:
      (A) LENGTH: 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: US4
   (xi) SEQUENCE DESCRIPTION: SEQ ID NO: 26
   GACAGCCCTA GTGGTATCGC AGTTACTCCG GATCCCACAA    40
   GCCGTCATGG ATATGGTGGC GGGGGCCCAC TGGGGAGTCC    80
   TGGCGGGCCT TGCCTACTAT                         100
(2) INFORMATION FOR SEQ ID NO: 27
   (i) SEQUENCE CHARACTERISTICS:
      (A) LENGTH: 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: ARG2
   (xi) SEQUENCE DESCRIPTION: SEQ ID NO: 27
   AGCAGCCCTA GTGGTGTCGC AGTTACTCCG GATCCCACAA    40
   AGCATCGTGG ACATGGTGGC GGGGGCCCAC TGGGGAGTCC    80
   TGGCGGGCCT TGCTTACTAT                         100
(2) INFORMATION FOR SEQ ID NO: 28
   (i) SEQUENCE CHARACTERISTICS:
      (A) LENGTH: 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: I15
   (xi) SEQUENCE DESCRIPTION: SEQ ID NO: 28
   GGCAGCCCTA GTGGTGTCGC AGTTACTCCG GATCCCGCAA    40
   GCTGTCGTGG ACATGGTGGC GGGGGCCCAC TGGGGAATCC    80
   TAGCGGGTCT TGCCTACTAT                         100
(2) INFORMATION FOR SEQ ID NO: 29
   (i) SEQUENCE CHARACTERISTICS:
      (A) LENGTH: 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: GH8
   (xi) SEQUENCE DESCRIPTION: SEQ ID NO: 29
   TGTGGGTATG GTGGTGGCGC ACGTCCTGCG TTTGCCCCAG    40
   ACCTTGTTCG ACATAATAGC CGGGGCCCAT TGGGGCATCT    80
   TGGCGGGCTT GGCCTATTAC                         100
(2) INFORMATION FOR SEQ ID NO: 30
   (i) SEQUENCE CHARACTERISTICS:
      (A) LENGTH: 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: I4
   (xi) SEQUENCE DESCRIPTION: SEQ ID NO: 30
   TGTGGGTATG GTGGTAGCAC ACGTCCTGCG TCTGCCCCAG    40
   ACCTTGTTCG ACATAATAGC CGGGGCCCAT TGGGGCATCT    80
   TGGCAGGCCT AGCCTATTAC                         100
(2) INFORMATION FOR SEQ ID NO: 31
   (i) SEQUENCE CHARACTERISTICS:
      (A) LENGTH: 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: I11
   (xi) SEQUENCE DESCRIPTION: SEQ ID NO: 31
   TGTGGGTATG GTGGTGGCGC AAGTCCTGCG TTTGCCCCAG    40
   ACCTTGTTCG ACGTGCTAGC CGGGGCCCAT TGGGGCATCT    80
   TGGCGGGCCT GGCCTATTAC                         100
(2) INFORMATION FOR SEQ ID NO: 32
   (i) SEQUENCE CHARACTERISTICS:
      (A) LENGTH: 100 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE;
      (C) INDIVIDUAL ISOLATE: I10
   (xi) SEQUENCE DESCRIPTION: SEQ ID NO: 32
   TACCACTATG CTCCTGGCAT ACTTGGTGCG CATCCCGGAG    40
   GTCATCCTGG ACATTATCAC GGGAGGACAC TGGGGCGTGA    80
   TGTTTGGCCT GGCTTATTTC                         100
(2) INFORMATION FOR SEQ ID NO: 33
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE: (ATCC # 40394)
      (C) INDIVIDUAL ISOLATE: hcv1
   (Xi) SEQUENCE DESCRIPTION: SEQ ID NO: 33
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCGTGCCCC CGCAAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 34
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: us5
   (xi) SEQUENCE DESCRIPTION: SEQ ID NO: 34
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCGTGCCCC CGCAAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 35
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (Vi) ORIGINAL SOURCE:
      (C) INDIVIDUAL ISOLATE: aus1
   (xi) SEQUENCE DESCRIPTION: SEQ ID NO: 35
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCACGCCCC CGCAAGATCA   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 36
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: sp2
   (xi) SEQUENCE DESCRIPTION: SEQ ID NO: 36
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATAAACCC   120
   GCTCAATGCC TGGAGATTTG GGCGTGCCCC CGCGAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 37
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: gm2
   (xi) SEQUENCE DESCRIPTION: SEQ ID NO: 37
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCGTGCCCC CGCAAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 38
   (i) SEQUENCE CHARACTERISTICS:
      (A) LENGTH: 252 nucleotides
      (B) TYPE: nucleic acid
      (C) STRANDEDKESS: single
      (D) TOPOLOGY: linear
   (ii) MOLECULE TYPE: DNA
   (vi) ORIGINAL SOURCE:
      (C) INDIVIDUAL ISOLATE: i21
   (xi) SEQUENCE DESCRIPTION: SEQ ID NO: 38
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATAAACCC   120
   GCTCAATGCC TGGAGATTTG GGCGTGCCCC CGCAAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 39
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: us4
   (xi) SEQUENCE DESCRIPTION: SEQ ID NO: 39
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCGTGCCCC CGCGAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 40
   (i) SEQUENCE CHARACTERISTICS;
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: jh1
   (xi) SEQUENCE DESCRIPTION: SEQ ID NO: 40
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCGTGCCCC CGCGAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA TC                                 252
(2) INFORMATION FOR SEQ ID NO: 41
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: nac5
   (xi) SEQUENCE DESCRIPTION: SEQ ID NO: 41
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCGTGCCCC CGCGAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 42
   (i) SEQUENCE CHARACTERISTICS:
      (A) LENGTH: 252 nucleotides
      (B) TYPE: nucleic acid
      (C) STRANDEDNESS; single
      (D) TOPOLOGY: linear
   (ii) MOLECULE TYPE: DNA
   (vi) ORIGINAL SOURCE:
      (C) INDIVIDUAL ISOLATE: arg2
   (xi) SEQUENCE DESCRIPTION: SEQ ID NO: 42
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCGTGCCCC CGCGAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 43
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: sp1
   (xi) SEQUENCE DESCRIPTION: SEQ ID NO: 43
   GTTAGTATGA GTGTCGTGCA GCCTCCAGGA CCCCCCCTCC    40
   CGGGAGAGCC ATAGTGGTCT GCGGAACCGG TGAGTACACC    80
   GGAATTGCCA GGACGACCGG GTCCTTTCTT GGATCAACCC   120
   GCTCAATGCC TGGAGATTTG GGCGTGCCCC CGCGAGACTG   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA CC                                 252
(2) INFORMATION FOR SEQ ID NO: 44
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: gh1
   (xi) SEQUENCE DESCRIPTION: SEQ ID NO: 44
   GTTAGTATGA GTGTCGTGCA GCCTCCAGGA CCCCCCCTCC     40
   CGGGAGAGCC ATAGTGGTCT GCGGAACCGG TGAGTACACC     80
   GGAATTGCCA GGACGACCGG GTCCTTTCTT GGATCAACCC    120
   GCTCAATGCC TGGAGATTTG GGCGTGCCCC CGCGAGACTG    160
   CTAGCCGAGT AGTGTTGGGT CGCGAAAGGC CTTGTGGTAC    200
   TGCCTGATAG GGTGCTTGCG AGTGCCCCGG GAGGTCTCGT    240
   AGACCGTGCA CC                                  252
(2) INFORMATION FOR SEQ ID NO: 45
   ( i ) SEQUENCE CHARACTERISTICS:
      (A) LENGTH; 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: i15
   (xi) SEQUENCE DESCRIPTION: SEQ ID NO: 45
(2) INFORMATION FOR SEQ ID NO: 46
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: i10
   (xi) SEQUENCE DESCRIPTION: SEQ ID NO: 46
   GCTAGTATCA GTGTCGTACA GCCTCCAGGC CCCCCCCTCC    40
   CGGGAGAGCC ATAGTGGTCT GCGGAACCGG TGAGTACACC    80
   GGAATTGCCG GGAAGACTGG GTCCTTTCTT GGATAAACCC   120
   ACTCTATGCC CGGCCATTTG GGCGTGCCCC CGCAAGACTG   160
   CTAGCCGAGT AGCGTTGGGT TGCGAAAGGC CTTGTGGTAC   200
   TGCCTGATAG GGTGCTTGCG AGTGCCCCGG GAGGTCTCGT   240
   AGACCGTGCA TC                                 252
(2) INFORMATION FOR SEQ ID NO: 47
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: arg6
   (xi) SEQUENCE DESCRIPTION: SEQ ID NO: 47
   GTTAGTATGA GTCTCGTACA GCCTCCAGGC CCCCCCCTCC    40
   CGGGAGAGCC ATAGTGGTCT GCGGAACCGG TGAGTACACC    80
   GGAATTGCTG GGAAGACTGG GTCCTTTCTT GGATAAACCC   120
   ACTCTATGCC CAGCCATTTG GGCGTGCCCC CGCAAGACTG   160
   CTAGCCGAGT AGCGTTGGGT TGCGAAAGGC CTTGTGGTAC    200
   TGCCTGATAG GGTGCTTGCG AGTGCCCCGG GAGGTCTCGT    240
   AGACCGTGCA TC                                  252
(2) INFORMATION FOR SEQ ID NO: 48
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: s21
   (xi) SEQUENCE DESCRIPTION: SEQ ID NO: 48
   GTTAGTACGA GTGTCGTGCA GCCTCCAGGA CTCCCCCTCC     40
   CGGGAGAGCC ATAGTGGTCT GCGGAACCGG TGAGTACACC     80
   GGAATCGCTG GGGTGACCGG GTCCTTTCTT GGAGCAACCC    120
   GCTCAATACC CAGAAATTTG GGCGTGCCCC CGCGAGATCA    160
   CTAGCCGAGT AGTGTTGGGT CGCGAAAGGC CTTGTGGTAC    200
   TGCCTGATAG GGTGCTTGCG AGTGCCCCGG GAGGTCTCGT    240
   AGACCGTGCA AC                                  252
(2) INFORMATION FOR SEQ ID NO: 49
   (i) SEQUENCE CHARACTERISTICS:
      (A) LENGTH: 252 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: gj61329
   (xi) SEQUENCE DESCRIPTION: SEQ ID NO: 49
   GTTAGTACGA GTGTCGTGCA GCCTCCAGGA CCCCCCCTCC    40
   CGGGAGAGCC ATAGTGGTCT GCGGAACCGG TGAGTACACC    80
   GGAATCGCTG GGGTGACCGG GTCCTTTCTT GGAGTAACCC   120
   GCTCAATACC CAGAAATTTG GGCGTGCCCC CGCGAGATCA   160
   CTAGCCGAGT AGTGTTGGGT CGCGAAAGGC CTTGTGGTAC   200
   TGCCTGATAG GGTGCTTGCG AGTGCCCCGG GAGGTCTCGT   240
   AGACCGTGCA AC                                 252
(2) INFORMATION FOR SEQ ID NO: 50
   (i) SEQUENCE CHARACTERISTICS:
      (A) LENGTH: 180 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: sa3
   (xi) SEQUENCE DESCRIPTION: SEQ ID NO: 50
   GTTAGTATGA GTGTCGAACA GCCTCCAGGA CCCCCCCTCC    40
   CGGGAGAGCC ATAGTGGTCT GCGGAACCGG TGAGTACACC    80
   GGAATTGCCG GGATGACCGG GTCCTTTCTT GGATAAACCC   120
   GCTCAATGCC CGGAGATTTG GGCGTGCCCC CGCGAGACTG   160
   CTAGCCGAGT AGTGTTGGGT                         180
(2) INFORMATION FOR SEQ ID NO; 51
   (i) SEQUENCE CHARACTERISTICS:
      (A) LENGTH: 180 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: sa4
   (xi) SEQUENCE DESCRIPTION: SEQ ID NO: 51
(2) INFORMATION FOR SEQ ID NO: 52
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE: (ATCC # 40394)
      (C) INDIVIDUAL ISOLATE: hcv1
   (xi) SEQUENCE DESCRIPTION: SEQ ID NO: 52
   ATGAGCACGA ATCCTAAACC TCAAAAAAAA AACAAACGTA    40
   ACACCAACCG TCGCCCACAG GACGTCAAGT TCCCGGGTGG    80
   CGGTCAGATC GTTGGTGGAG TTTACTTGTT GCCGCGCAGG   120
   GGCCCTAGAT TGGGTGTGCG CGCGACGAGA AAGACTTCCG   160
   AGCGGTCGCA ACCTCGAGGT AGACGTCAGC CTATCCCCAA   200
   GGCTCGTCGG CCCGAGGGCA GGACCTGGGC TCAGCCCGGG   240
   TACCCTTGGC CCCTCTATGG CAATGAGGGC TGCGGGTGGG   280
   CGGGATGGCT CCTGTCTCCC CGTGGCTCTC GGCCTAGCTG   320
   GGGCCCCACA GACCCCCGGC GTAGGTCGCG CAATTTGGGT   360
   AAGGTCATCG ATACCCTTAC GTGCGGCTTC GCCGACCTCA   400
   TGGGGTACAT ACCGCTCGTC GGCGCCCCTC TTGGAGGCGC   440
   TGCCAGGGCC CTGGCGCATG GCGTCCGGGT TCTGGAAGAC   480
   GGCGTGAACT ATGCAACAGG GAACCTTCCT GGTTGCTCTT   520
   TCTCTATCTT CCTTCTGGCC CTGCTCTCT               549
(2) INFORMATION FOR SEQ ID NO: 53
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: us5
   (xi) SEQUENCE DESCRIPTION: SEQ ID NO: 53
   ATGAGCACGA ATCCTAAACC TCAAAGAAAA ACCAAACGTA    40
   ACACCAACCG TCGCCCACAG GACGTCAAGT TCCCGGGTGG    80
   CGGTCAGATC GTTGGTGGAG TTTACTTGTT GCCGCGCAGG   120
   GGCCCTAGAT TGGGTGTGCG CGCGACGAGG AAGACTTCCG   160
   AGCGGTCGCA ACCTCGAGGT AGACGTCAGC CTATCCCCAA   200
   GGCGCGTCGG CCCGAGGGCA GGACCTGGGC TCAGCCCGGG   240
   TACCCTTGGC CCCTCTATGG CAATGAGGGT TGCGGGTGGG   280
   CGGGATGGCT CCTGTCTCCC CGTGGCTCTC GGCCTAGTTG   320
   GGGCCCCACA GACCCCCGGC GTAGGTCGCG CAATTTGGGT   360
   AAGGTCATCG ATACCCTTAC GTGCGGCTTC GCCGACCACA   400
   TGGGGTACAT ACCGCTCGTC GGCGCCCCTC TTGGAGGCGC   440
   TGCCAGGGCT CTGGCGCATG GCGTCCGGGT TCTGGAAGAC   480
   GGCGTGAACT ATGCAACAGG GAACCTTCCT GGTTGCTCTT   520
   TCTCTATCTT CCTTCTGGCC CTGCTCTCT               549
(2) INFORMATION FOR SEQ ID NO: 54
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: aus1
   (xi) SEQUENCE DESCRIPTION: SEQ ID NO: 54
   ATGAGCACGA ATCCTAAACC TCAAAGAAAA ACCAAACGTA    40
   ACACCAACCG TCGCCCACAG GACGTTAAGT TCCCGGGTGG    80
   CGGTCAGATC GTTGGTGGAG TTTACTTGTT GCCGCGCAGG   120
   GGCCCTAGAT TGGGTGTGCG CGCGACGAGG AAGACTTCCG   160
   AGCGGTCGCA ACCTCGAGGT AGACGTCAGC CTATCCCTAA   200
   GGCGCGTCGG CCCGAGGGCA GGACCTGGGC TCAGCCCGGG   240
   TACCCCTGGC CCCTCTATGG TAATGAGGGT TGCGGATGGG   280
   CGGGATGGCT CCTGTCCCCC CGTGGCTCTC GGCCTAGTTG   320
   GGGCCCTACA GACCCCCGGC GTAGGTCGCG CAATTTGGGT   360
   AAGGTCATCG ATACCCTCAC GTGCGGCTTC GCCGACCACA   400
   TGGGGTACAT TCCGCTCGTT GGCGCCCCTC TTGGGGGCGC   440
   TGCCAGGGCC CTGGCGCATG GCGTCCGGGT TCTGGAAGAC   480
   GGCGTGAACT ATGCAACAGG GAATCTTCCT GGTTGCTCTT   520
   TCTCTATCTT CCTTCTGGCC CTTCTCTCT               549
(2) INFORMATION FOR SEQ ID NO: 55
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: sp2
   (xi) SEQUENCE DESCRIPTION: SEQ ID NO: 55
   ATGAGCACGA ATCCTAAACC TCAAAGAAAA ACCAAACGTA    40
   ACACCAACCG TCGCCCACAG GACGTCAAGT TCCCGGGTGG    80
   CGGTCAGATC GTTGGTGGAG TTTACTTGTT GCCGCGCAGG   120
   GGCCCTAGAT TGGGTGTGCG CACGACGAGG AAGACTTCCG   160
   AGCGGTCGCA ACCTCGAGGT AGACGTCAGC CCATCCCCAA   200
   GGCTCGTCGA CCCGAGGGCA GGACCTGGGC TCAGCCCGGG   240
   TACCCTTGGC CCCTCTATGG CAATGAGGGC TGCGGGTGGG   280
   CGGGATGGCT CCTGTCTCCC CGTGGCTCTC GGCCTAGCTG   320
   GGGCCCCACA GACCCCCGGC GTAGGTCGCG CAATTTGGGT   360
   AAGGTCATCG ATACCCTTAC GTGCGGCTTC GCCGACCTCA   400
   TGGGGTACAT ACCGCTCGTC GGCGCCCCTC TTGGAGGCGC   440
   TGCCAGAGCC CTGGCGCATG GCGTCCGGGT TCTGGAAGAC   480
   GGCGTGAACT ATGCAACAGG GAACCTTCCC GGTTGCTCTT   520
   TCTCTATCTT CCTTCTGGCC CTGCTCTCT               549
(2) INFORMATION FOR SEQ ID NO: 56
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: gm2
   (xi) SEQUENCE DESCRIPTION: SEQ ID NO: 56
   ATGAGCACGA ATCCTAAACC TCAAAGAAGA ACCAAACGTA    40
   ACACCAACCG TCGCCCACAG GACGTCAAGT TCCCGGGTGG    80
   CGGTCAGATC GTTGGTGGAG TTTACTTGTT GCCGCGCAGG   120
   GGCCCTAGAT TGGGTGTGCG CGCGACGAGG AAGACTTCCG   160
   AGCGGTCGCA ACCTCGAGGT AGACGTCAGC CTATCCCCAA   200
   GGCACGTCGG CCCGAGGGTA GGACCTGGGC TCAGCCCGGG   240
   TACCCTTGGC CCCTCTATGG CAATGAGGGT TGCGGGTGGG   280
   CGGGATGGCT CCTGTCTCCC CGCGGCTCTC GGCCTAACTG   320
   GGGCCCCACA GACCCCCGGC GTAGGTCGCG CAATTTGGGT   360
   AAGGTCATCG ATACCCTTAC GTGCGGCTTC GCCGACCTCA   400
   TGGGGTACAT ACCGCTCGTC GGCGCCCCTC TTGGAGGCGC   440
   TGCCAGGGCC CTGGCGCATG GCGTCCGGGT TCTGGAAGAC   480
   GGCGTGAACT ATGCAACAGG GAACCTTCCT GGTTGCTCTT   520
   TCTCTATCTT CCTTCTGGCC CTGCTCTCT               549
(2) INFORMATION FOR SEQ ID NO: 57
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: i21
   (xi) SEQUENCE DESCRIPTION: SEQ ID NO: 57
   ATGAGCACGA ATCCTAAACC TCAAAGAAAA ACCAAACGTA    40
   ACACCAACCG TCGCCCACAG GACGTCAAGT TCCCGGGTGG    80
   CGGTCAGATC GTTGGTGGAG TTTACTTGTT GCCGCGCAGG   120
   GGCCCTAGAT TGGGTGTGCG CGCGACGAGG AAGACTTCCG   160
   AGCGGTCGCA ACCTCGTGGT AGACGCCAGC CTATCCCCAA   200
   GGCGCGTCGG CCCGAGGGCA GGACCTGGGC TCAGCCCGGG   240
   TACCCTTGGC CCCTCTATGG CAATGAGGGT TGCGGGTGGG   280
   CGGGATGGCT CCTGTCTCCC CGTGGCTCTC GGCCTAGCTG   320
   GGGCCCCACA GACCCCCGGC GTAGGTCGCG CAATTTGGGT   360
   AAGGTCATCG ATACCCTTAC GTGCGGCTTC GCCGACCTCA   400
   TGGGGTACAT ACCGCTCGTC GGCGCCCCTC TTGGAGGCGC   440
   TGCCAGGGCC CTGGCGCATG GCGTCCGGGT TCTGGAAGAC   480
   GGCGTGAACT ATGCAACAGG GAACCTTCCT GGTTGCTCTT   520
   TTTCTATTTT CCTTCTGGCC CTGCTCTCT               549
(2) INFORMATION FOR SEQ ID NO: 58
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: us4
   (xi) SEQUENCE DESCRIPTION: SEQ ID NO: 58
   ATGAGCACGA ATCCTAAACC TCAAAGAAAA ACCAAACGTA    40
   ACACCAACCG CCGCCCACAG GACGTTAAGT TCCCGGGCGG    80
   TGGCCAGGTC GTTGGTGGAG TTTACCTGTT GCCGCGCAGG   120
   GGCCCCAGGT TGGGTGTGCG CGCGACTAGG AAGACTTCCG   160
   AGCGGTCGCA ACCTCGTGGA AGGCGACAAC CTATCCCCAA   200
   GGCTCGCCAG CCCGAGGGCA GGGCCTGGGC TCAGCCCGGG   240
   TACCCTTGGC CCCTCTATGG CAATGAGGGT ATGGGGTGGG   280
   CAGGATGGCT CCTGTCACCC CGTGGCTCTC GGCCTAGTTG   320
   GGGCCCCACG GACCCCCGGC GTAGGTCGCG TAATTTGGGT   360
   AAGGTCATCG ATACCCTCAC ATGCGGCTTC GCCGACCTCA   400
   TGGGGTACAT TCCGCTCGTC GGCGCCCCCC TTAGGGGCGC   440
   TGCCAGGGCC TTGGCGCATG GCGTCCGGGT TCTGGAGGAC   480
   GGCGTGAACT ACGCAACAGG GAATCTGCCC GGTTGCTCCT   520
   TTTCTATCTT CCTCTTGGCT CTGCTGTCC               549
(2) INFORMATION FOR SEQ ID NO: 59
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE; jh1
   (xi) SEQUENCE DESCRIPTION: SEQ ID NO: 59
   ATGAGCACAA ATCCTAAACC TCAAAGAAAA ACCAAACGTA     40
   ACACCAACCG CCGCCCACAG GACGTCAAGT TCCCGGGCGG     80
   TGGTCAGATC GTTGGTGGAG TTTACCTGTT GCCGCGCAGG    120
   GGCCCCAGGT TGGGTGTGCG CGCGACTAGG AAGACTTCCG    160
   AGCGGTCGCA ACCTCGTGGA AGGCGACAAC CTATCCCCAA    200
   GGCTCGCCAG CCCGAGGGCA GGGCCTGGGC TCAGCCCGGG    240
   TACCCTTGGC CCCTCTATGG CAACGAGGGT ATGGGGTGGG    280
   CAGGATGGCT CCTGTCACCC CGTGGCTCTC GGCCTAGTTG    320
   GGGCCCCACG GACCCCCGGC GTAGGTCGCG TAATTTGGGT    360
   AAGGTCATCG ATACCCTCAC ATGCGGCTTC GCCGACCTCA    400
   TGGGGTACAT TCCGCTTGTC GGCGCCCCCC TAGGGGGCGC    440
   TGCCAGGGCC CTGGCACATG GTGTCCGGGT TCTGGAGGAC    480
   GGCGTGAACT ATGCAACAGG GAATTTGCCC GGTTGCTCTT    520
   TCTCTATCTT CCTCTTGGCT CTGCTGTCC                549
(2) INFORMATION FOR SEQ ID NO: 60
   (i) SEQUENCE CHARACTERISTICS;
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: nac5
   (xi) SEQUENCE DESCRIPTION: SEQ ID NO: 60
   ATGAGCACAA ATCCTAAACC CCAAAGAAAA ACCAAACGTA    40
   ACACCAACCG TCGCCCACAG GACGTCAAGT TCCCGGGCGG    80
   TGGTCAGATC GTTGGTGGAG TTTACCTGTT GCCGCGCAGG   120
   GGCCCCAGGT TGGGTGTGCG CGCGACTAGG AAGACTTCCG   160
   AGCGGTCGCA ACCTCGTGGA AGGCGACAAC CTATCCCCAA   200
   GGCTCGCCGG CCCGAGGGCA GGTCCTGGGC TCAGCCCGGG   240
   TACCCTTGGC CCCTCTATGG CAACGAGGGT ATGGGGTGGG   280
   CAGGATGGCT CCTGTCACCC CGCGGCTCCC GGCCTAGTTG   320
   GGGCCCCACG GACCCCCGGC GTAGGTCGCG TAATTTGGGT   360
   AAGGTCATCG ATACCCTCAC ATGCGGCTTC GCCGACCTCA   400
   TGGGGTACAT TCCGCTCGTC GGCGCCCCCC TAGGGGGCGC   440
   TGCCAGGGCC CTGGCACATG GTGTCCGGGT TCTGGAGGAC   480
   GGCGTGAACT ATGCAACAGG GAATTTGCCT GGTTGCTCTT   520
   TCTCTATCTT CCTCTTGGCT CTGCTGTCC               549
(2) INFORMATION FOR SEQ ID NO: 61
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: arg2
   (xi) SEQUENCE DESCRIPTION: SEQ ID NO: 61
   ATGAGCACGA ATCCTAAACC TCAAAGAAAA ACCAAACGTA     40
   ACACCAACCG CCGCCCACAG GACGTCAAGT TCCCGGGCGG     80
   TGGTCAGATC GTTGGTGGAG TTTACTTGTT GCCGCGCAGG    120
   GGCCCCAGGT TGGGTGTGCG CGCGACTAGG AAGACTTCCG    160
   AGCGGTCGCA ACCTCGTGGA AGGCGACAAC CTATCCCCAA    200
   GGCTCGCCAG CCCGAGGGTA GGGCCTGGGC TCAGCCCGGG    240
   TACCCTTGGC CCCTCTATGG CAATGAGGGT ATGGGGTGGG    280
   CAGGGTGGCT CCTGTCCCCC CGCGGCTCCC GGCCTAGTTG    320
   GGGCCCCACA GACCCCCGGC GTAGGTCGCG TAATTTGGGT    360
   AAGGTCATCG ATACCCTCAC ATGCGGCTTC GCCGACCTCA    400
   TGGGGTACAT TCCGCTCGTC GGCGCCCCCC TAGGGGGCGC    440
   TGCCAGGGCC CTGGCGCATG GCGTCCGGGT TCTGGAGGAC    480
   GGCGTGAACT ATGCAACAGG GAATCTGCCC GGTTGCTCTT    520
   TCTCTATCTT CCTCTTGGCT TTGCTGTCC                549
(2) INFORMATION FOR SEQ ID NO: 62
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: sp1
   (xi) SEQUENCE DESCRIPTION: SEQ ID NO: 62
   ATGAGCACGA ATCCTAAACC TCAAAGAAAA ACCAAACGTA     40
   ACACCAACCG CCGCCCACAG GACGTCAAGT TCCCGGGCGG     80
   TGGTCAGATC GTTGGTGGAG TTTACCTGTT GCCGCGCAGG    120
   GGCCCCAGGT TGGGTGTGCG CGCGACTAGG AAGACTTCCG    160
   AGCGGTCGCA ACCTCGTGGA AGGCGACAAC CTATCCCCAA    200
   GGCTCGCCGG CCCGAGGGCA GGGCCTGGGC TCAGCCCGGG    240
   TATCCTTGGC CCCTCTATGG CAATGAGGGT CTGGGGTGGG    280
   CAGGATGGCT CCTGTCACCC CGCGGCTCTC GGCCTAGCTG    320
   GGGCCCTACC GACCCCCGGC GTAGGTCGCG CAACTTGGGT    360
   AAGGTCATCG ATACCCTTAC GTGCGGCTTC GCCGACCTCA    400
   TGGGGTACAT TCCGCTCGTC GGCGCCCCCC TTAGGGGCGC    440
   TGCCAGGGCC CTGGCGCATG GCGTCCGGGT TCTGGAGGAC    480
   GGCGTGAACT ATGCAACAGG GAATTTGCCC GGTTGCTCTT    520
   TCTCTATCTT CCTCTTGGCT TTGCTGTCC                549
(2) INFORMATION FOR SEQ ID NO: 63
   (i) SEQUENCE CHARACTERISTICS:
      (A) LENGTH: 549 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: gh1
   (xi) SEQUENCE DESCRIPTION: SEQ ID NO: 63
   ATGAGCACGA ATCCTAAACC TCAAAGAAAA ACCAAACGTA     40
   ACACCAACCG CCGCCCACAG GACGTCAAGT TCCCGGGCGG     80
   TGGTCAGATC GTTGGTGGAG TTTACTTGTT GCCGCGCAGG    120
   GGCCCCAGGT TGGGTGTGCG CGCGACTAGG AAGACTTCCG    160
   AGCGGTCGCA ACCTCGTGGA AGGCGACAAC CTATCCCCAA    200
   GGCTCGCCGG CCCGAGGGCA GGGCCTGGGC TCAGCCCGGG    240
   TACCCTTGGC CCCTCTATGG CAATGAGGGT ATGGGGTGGG    280
   CAGGATGGCT CCTGTCACCC CGTGGTTCTC GGCCTAGTTG    320
   GGGCCCCACG GACCCCCGGC GTAGGTCGCG CAATTTGGGT    360
   AAGATCATCG ATACCCTCAC GTGCGGCTTC GCCGACCTCA    400
   TGGGGTACAT TCCGCTCGTC GGCGCCCCCC TAGGGGGCGC    440
   TGCCAGGGCC CTGGCGCATG GCGTCCGGGT TCTGGAGGAC    480
   GGCGTGAACT ATGCAACAGG GAATCTGCCC GGTTGCTCCT    520
   TTTCTATCTT CCTTCTGGCT TTGCTGTCC                549
(2) INFORMATION FOR SEQ ID NO: 64
   (i) SEQUENCE CHARACTERISTICS:
      (A) LENGTH: 549 nucleotides
      (B) TYPE:, nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: i15
   (xi) SEQUENCE DESCRIPTION: SEQ ID NO: 64
   ATGAGCACGA ATCCTAAACC TCAAAGAAAA ACCAAACGTA    40
   ACACCAACCG CCGCCCACAG GACGTCAAGT TCCCGGGCGG    80
   TGGTCAGATC GTTGGTGGAG TTTACCTGTT GCCGCGCAGG   120
   GGCCCCAGGT TGGGTGTGCG CGCGACTAGG AAGACTTCCG   160
   AGCGGTCGCA ACCTCGTGGA AGGCGACAAC CTATCCCCAA   200
   GGCTCGCCAG CCCGAGGGCA GGGCCTGGGC TCAGCCCGGG   240
   TACCCCTGGC CCCTCTATGG CAATGAGGGT ATGGGGTGGG   280
   CAGGATGGCT CCTGTCACCC CGCGGCTCCC GGCCTAGTTG   320
   GGGCCCCAAA GACCCCCGGC GTAGGTCGCG TAATTTGGGT   360
   AAGGTCATCG ATACCCTCAC ATGCGGCTTC GCCGACCTCA   400
   TGGGGTACAT TCCGCTCGTC GGCGCCCCCT TAGGGGGCGC   440
   TGCCAGGGCC CTGGCGCATG GCGTCCGGGT TCTGGAGGAC   480
   GGCGTGAACT ATGCAACAGG GAATCTACCC GGTTGCTCTT   520
   TCTCTATCTT CCTCTTGGCT TTGCTGTCC              549
(2) INFORMATION FOR SEQ ID NO: 65
   (i) SEQUENCE CHARACTERISTICS:
      (A) LENGTH: 549 nucleotides
      (B) TYPE: nucleic acid
      (C) STRANDEDNESS : single
      (D) TOPOLOGY: linear
   (ii) MOLECULE TYPE: DNA
   (vi) ORIGINAL SOURCE:
      (C) INDIVIDUAL ISOLATE: i10
   (xi) SEQUENCE DESCRIPTION: SEQ ID NO: 65
   ATGAGCACAA ATCCTAAACC TCAAAGAAAA ACCAAAAGAA    40
   ACACTAACCG CCGCCCACAG GACGTCAAGT TCCCGGGCGG    80
   TGGCCAGATC GTTGGCGGAG TATACTTGCT GCCGCGCAGG   120
   GGCCCGAGAT TGGGTGTGCG CGCGACGAGG AAAACTTCCG   160
   AACGATCCCA GCCACGCGGA AGGCGTCAGC CCATCCCTAA   200
   AGATCGTCGC ACCGCTGGCA AGTCCTGGGG AAGGCCAGGA   240
   TATCCTTGGC CCCTGTATGG GAATGAGGGT CTCGGCTGGG   280
   CAGGGTGGCT CCTGTCCCCC CGTGGCTCTC GCCCTTCATG   320
   GGGCCCCACT GACCCCCGGC ATAGATCGCG CAACTTGGGT   360
   AAGGTCATCG ATACCCTAAC GTGCGGTTTT GCCGACCTCA   400
   TGGGGTACAT TCCCGTCATC GGCGCCCCCG TTGGAGGCGT   440
   TGCCAGAGCT CTCGCCCACG GAGTGAGGGT TCTGGAGGAT   480
   GGGGTAAATT ATGCAACAGG GAATTTGCCC GGTTGCTCTT   520
   TCTCTATCTT TCTCTTAGCC CTCTTGTCT               549
(2) INFORMATION FOR SEQ ID NO: 66
   (i) SEQUENCE CHARACTERISTICS:
      (A) LENGTH: 510 nucleotides
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (C) INDIVIDUAL ISOLATE: arg6
   (xi) SEQUENCE DESCRIPTION: SEQ ID NO: 66
   ATGAGCACAA ATCCTCAACC TCAAAGAAAA ACCAAAAGAA    40
   ACACTAACCG CCGCCCACAG GACGTCAAGT TCCCGGGCGG    80
   TGGTCAGATC GTTGGCGGAG TATACTTGTT GCCGCGCAGG   120
   GGCCCCAGGT TGGGTGTGCG CGCGACGAGG AAAACTTCCG   160
   AACGGTCCCA GCCACGTGGG AGGCGCCAGC CCATCCCCAA   200
   AGATCGGCGC ACCACTGGCA AGTCCTGGGG GAAGCCAGGA   240
   TACCCTTGGC CCCTGTATGG GAATGAGGGT CTCGGCTGGG   280
   CAGGGTGGCT CCTGTCCCCC CGCGGTTCTC GCCCTTCATG   320
   GGGCCCCACT GACCCCCGGC ATAGATCACG CAACTTGGGT   360
   AAGGTCATCG ATACCCTAAC GTGTGGTTTT GCCGACCTCA   400
   TGGGGTACAT TCCCGTCGGT GGTGCCCCCG TTGGTGGTGT   440
   CGCCAGAGCC CTTGCCCATG GGGTGAGGGT TCTGGAAGAC   480
   GGGATAAATT ATGCAACAGG GAATCTGCCC              510
(2) INFORMATION FOR SEQ ID NO: 67
   (i) SEQUENCE CHARACTERISTICS:
      (A) LENGTH: 29 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 67
   CAAACGTAAC ACCAACCGRC GCCCACAGG    29
(2) INFORMATION FOR SEQ ID NO: 68
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 68
   ACAGAYCCGC AKAGRTCCCC CACG    24
(2) INFORMATION FOR SEQ ID NO: 69
   (i) SEQUENCE CHARACTERISTICS:
      (A) LENGTH: 30 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 69
   CGAACCTCGA GGTAGACGTC AGCCTATCCC 30
(2) INFORMATION FOR SEQ ID NO: 70
   (i) SEQUENCE CHARACTERISTICS:
      (A) LENGTH: 30 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 70
   GCAACCTCGT GGAAGGCGAC AACCTATCCC 30
(2) INFORMATION FOR SEQ ID NO: 71
   (i) SEQUENCE CHARACTERISTICS:
      (A) LENGTH: 30 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 71
   GTCACCAATG ATTGCCCTAA CTCGAGTATT 30
(2) INFORMATION FOR SEQ ID NO: 72
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 72
   GTCACGAACG ACTGCTCCAA CTCAAG 26
(2) INFORMATION FOR SEQ ID NO: 73
   (i) SEQUENCE CHARACTERISTICS:
      (A) LENGTH: 2B nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 73
(2) INFORMATION FOR SEQ ID NO: 74
   (i) SEQUENCE CHARACTERISTICS:
      (A) LENGTH: 28 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 74
   TGGAYATGGT GGYGGGGGCY CACTGGGG 28
(2) INFORMATION FOR SEQ ID NO: 75
   (i) SEQUENCE CHARACTERISTICS:
      (A) LENGTH: 20 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 75
   ATGATGAACT GGTCVCCYAC 20
(2) INFORMATION FOR SEQ ID NO: 76
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 76
   ACCTTVGCCC AGTTSCCCRC CATGGA 26
(2) INFORMATION FOR SEQ ID NO: 77
   (i) SEQUENCE CHARACTERISTICS:
      (A) LENGTH: 22 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 77
   AACCCACTCT ATGYCCGGYC AT 22
(2) INFORMATION FOR SEQ ID NO: 78
   (i) SEQUENCE CHARACTERISTICS:
      (A) LENGTH: 18 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 78
   GAATCGCTGG GGTGACCG 18
(2) INFORMATION FOR SEQ ID NO: 79
   (i) SEQUENCE CHARACTERISTICS:
      (A) LENGTH: 28 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 75
(2) INFORMATION FOR SEQ ID NO: 80
   (i) SEQUENCE CHARACTERISTICS:
      (A) LENGTH: 18 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 80
   TTGCGGGGGC ACGCCCAA 18
(2) INFORMATION FOR SEQ ID NO: 81
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 81
   YGAAGCGGGC ACAGTCARRC AAGARAGCAG GGC 33
(2) INFORMATION FOR SEQ ID NO: 82
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 82
   RTARAGCCCY GWGGAGTTGC GCACTTGGTR GGC 33
(2) INFORMATION FOR SEQ ID NO: 83
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 83
   RATACTCGAG TTAGGGCAAT CATTGGTGAC RTG 33
(2) INFORMATION FOR SEQ ID NO: 84
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (Xi) SEQUENCE DESCRIPTION: SEQ ID NO: 84
   AGYRTGCAGG ATGGYATCRK BCGYCTCGTA CAC 33
(2) INFORMATION FOR SEQ ID NO; 85
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 85
(2) INFORMATION FOR SEQ ID NO: 86
   (i) SEQUENCE E CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 86
   CGTRGGGGTY AYCGCCACCC AACACCTCGA GRC 33
(2) INFORMATION FOR SEQ ID NO: 87
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 87
   CGTYGYGGGG AGTTTGCCRT CCCTGGTGGC YAC 33
(2) INFORMATION FOR SEQ ID NO: 88
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 88
   CCCGACAAGC AGATCGATGT GACGTCGAAG CTG 33
(2) INFORMATION FOR SEQ ID NO: 89
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 89
   CCCCACGTAG ARGGCCGARC AGAGRGTGGC GCY 33
(2) INFORMATION FOR SEQ ID NO: 90
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (Xi) SEQUENCE DESCRIPTION: SEQ ID NO: 90
   YTGRCCGACA AGAAAGACAG ACCCGCAYAR GTC 33
(2) INFORMATION FOR SEQ ID NO: 91
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 91
   CGTCCAGTGG YGCCTGGGAG AGAAGGTGAA CAG 33
(2) INFORMATION FOR SEQ ID NO: 92
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 92
   GCCGGGATAG ATRGARCAAT TGCARYCTTG CGT 33
(2) INFORMATION FOR SEQ ID NO: 93
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 93
   CATATCCCAT GCCATGCGGT GACCCGTTAY ATG 33
(2) INFORMATION FOR SEQ ID NO: 94
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 94
   YACCAAYGCC GTCGTAGGGG ACCARTTCAT CAT 33
(2) INFORMATION FOR SEQ ID NO: 95
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (Xi) SEQUENCE DESCRIPTION: SEQ ID NO: 95
   GATGGCTTGT GGGATCCGGA GYASCTGAGC YAY 33
(2) INFORMATION FOR SEQ ID NO: 96
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 96
   GACTCCCCAG TGRGCWCCAG CGATCATRTC CAW 33
(2) INFORMATION FOR SEQ ID NO: 97
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 97
   CCCCACCATG GAGAAATACG CTATGCCCGC YAG 33
(2) INFORMATION FOR SEQ ID NO: 98
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 98
   TAGYAGCAGY ACTACYARGA CCTTCGCCCA GTT 33
(2) INFORMATION FOR SEQ ID NO: 99
   (i) SEQUENCE CHARACTERISTICS:
      (A) LENGTH: 33 nucleotides
      (B) TYPE: nucleic acid
      (C) STRANDEDNESS; single
      (D) TOPOLOGY: linear
   (ii) MOLECULE TYPE: DNA
   (xi) SEQUENCE DESCRIPTION: SEQ ID NO: 99
   GSTGACGTGR GTKTCYGCGT CRACGCCGGC RAA 33
(2) INFORMATION FOR SEQ ID NO: 100
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 100
   GGAAGYTGGG ATGGTYARRC ARGASAGCAR AGC 33
(2) INFORMATION FOR SEQ ID NO: 101
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 101
   GTAYAYYCCG GACRCGTTGC GCACTTCRTA AGC 33
(2) INFORMATION FOR SEQ ID NO: 102
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 102
   AATRCTTGMG TTGGAGCART CGTTYGTGAC ATG 33
(2) INFORMATION FOR SEQ ID NO: 103
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 103
   RGYRTGCATG ATCAYGTCCG YYGCCTCATA CAC 33
(2) INFORMATION FOR SEQ ID NO: 104
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 104
   RTTGTYYTCC CGRACGCARG GCACGCACCC RGG 33
(2) INFORMATION FOR SEQ ID NO: 105
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 105
   CGTGGGRGTS AGCGCYACCC AGCARCGGGA GSW 33
(2) INFORMATION FOR SEQ ID NO: 106
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 106
   YGTRGTGGGG AYGCTGKHRT TCCTGGCCGC VAR 33
(2) INFORMATION FOR SEQ ID NO: 107
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 107
   CCCRACGAGC AARTCGACRT GRCGTCGTAW TGT 33
(2) INFORMATION FOR SEQ ID NO: 108
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 108
   YCCCACGTAC ATAGCSGAMS AGARRGYAGC CGY 33
(2) INFORMATION FOR SEQ ID NO: 109
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 109
   CTGGGAGAYR AGRAAAACAG ATCCGCARAG RTC 33
(2) INFORMATION FOR SEQ ID NO: 110
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 110
   YGTCTCRTGC CGGCCAGSBG AGAAGGTGAA YAG 33
(2) INFORMATION FOR SEQ ID NO: 111
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 111
   GCCGGGATAG AKKGAGCART TGCAKTCCTG YAC 33
(2) INFORMATION FOR SEQ ID NO: 112
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 112
   CATATCCCAA GCCATRCGRT GGCCTGAYAC CTG 33
(2) INFORMATION FOR SEQ ID NO: 113
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 113
   CACTARGGCT GYYGTRGGYG ACCAGTTCAT CAT 33
(2) INFORMATION FOR SEQ ID NO: 114
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 114
   GACRGCTTGT GGGATCCGGA GTAACTGCGA YAC 33
(2) INFORMATION FOR SEQ ID NO: 115
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 115
   GACTCCCCAG TGRGCCCCCG CCACCATRTC CAT 33
(2) INFORMATION FOR SEQ ID NO: 116
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 116
   SCCCACCATG GAWWAGTAGG CAAGGCCCGC YAG 33
(2) INFORMATION FOR SEQ ID NO: 117
   (i) SEQUENCE CHARACTERISTICS:
      (A) LENGTH: 33 nucleotides
      (B) TYPE: nucleic acid
      (C) STRANDEDNESS: single
      (D) TOPOLOGY; linear
   (ii) MOLECULE TYPE: DNA
   (xi) SEQUENCE DESCRIPTION: SEQ ID NO: 117
   GAGTAGCATC ACAATCAADA CCTTAGCCCA GTT 33
(2) INFORMATION FOR SEQ ID NO: 118
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 118
   YGWCRYGYRG GTRTKCCCGT CAACGCCGGC AAA 33
(2) INFORMATION FOR SEQ ID NO: 119
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (it) MOLECULE TYPE: DNA
   (xi) SEQUENCE DESCRIPTION: SEQ ID NO: 119
   TCCTCACAGG GGAGTGATTC ATGGTGGAGT GTC 33
(2) INFORMATION FOR SEQ ID NO: 120
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 120
   ATGGCTAGAC GCTTTCTGCG TGAAGACAGT AGT 33
(2) INFORMATION FOR SEQ ID NO: 121
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (it) MOLECULE TYPE: DNA
   (xi) SEQUENCE DESCRIPTION: SEQ ID NO: 121
   GCCTGGAGGC TGCACGRCAC TCATACTAAC GCC 33
(2) INFORMATION FOR SEQ ID NO: 122
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 122
   CGCAGACCAC TATGGCTCTY CCGGGAGGGG GGG 33
(2) INFORMATION FOR SEQ ID NO: 123
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 123
   TCRTCCYGGC AATTCCGGTG TACTCACCGG TTC 33
(2) INFORMATION FOR SEQ ID NO: 124
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (it) MOLECULE TYPE: DNA
   (xi) SEQUENCE DESCRIPTION: SEQ ID NO: 124
   GCATIGAGCG GGTTDATCCA AGAAAGGACC CGG 33
(2) INFORMATION FOR SEQ ID NO: 125
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 125
   AGCAGTCTYG CGGGGGCACG CCCAARTCTC CAG 33
(2) INFORMATION FOR SEQ ID NO: 126
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 126
   ACAAGGCCTT TCGCGACCCA ACACTACTCG GCT 33
(2) INFORMATION FOR SEQ ID NO: 127
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 127
   GGGGCACTCG CAAGCACCCT ATCAGGCAGT ACC 33
(2) INFORMATION FOR SEQ ID NO: 128
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 128
   YGTGCTCATG RTGCACGGTC TACGAGACCT CCC 33
(2) INFORMATION FOR SEQ ID NO; 129
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 129
(2) INFORMATION FOR SEQ ID NO: 130
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 130
   CGGGAACTTR ACGTCCTGTG GGCGRCGGTT GGT 33
(2) INFORMATION FOR SEQ ID NO: 131
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 131
   CARGTAAACT CCACCRACGA TCTGRCCRCC RCC 33
(2) INFORMATION FOR SEQ ID NO: 132
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 132
   RCGCACACCC AAYCTRGGGC CCCTGCGCGG CAA 33
(2) INFORMATION FOR SEQ ID NO; 133
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 133
(2) INFORMATION FOR SEQ ID NO: 134
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 134
   RCGHRCCTTG GGGATAGGCT GACGTCWACC TCG 33
(2) INFORMATION FOR SEQ ID NO: 135
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 135
   RCGHRCCTTG GGGATAGGTT GTCGCCWTCC ACG 33
(2) INFORMATION FOR SEQ ID NO: 136
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 136
   YCCRGGCTGR GCCCAGRYCC TRCCCTCGGR YYG 33
(2) INFORMATION FOR SEQ ID NO: 137
   (i) SEQUENCE CHARACTERISTICS;
      (A) LENGTH: 33 nucleotides
      (By TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 137
   BSHRCCCTCR TTRCCRTAGA GGGGCCADGG RTA 33
(2) INFORMATION FOR SEQ ID NO: 138
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 138
   GCCRCGGGGW GACAGGAGCC ATCCYGCCCA CCC 33
(2) INFORMATION FOR SEQ ID NO: 139
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 139
   CCGGGGGTCY GTGGGGCCCC AYCTAGGCCG RGA 33
(2) INFORMATION FOR SEQ ID NO: 140
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (it) MOLECULE TYPE: DNA
   (xi) SEQUENCE DESCRIPTION: SEQ ID NO: 140
   ATCGATGACC TTACCCAART TRCGCGACCT RCG 33
(2) INFORMATION FOR SEQ ID NO: 141
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 141
   CCCCATGAGR TCGGCGAAGC CGCAYGTRAG GGT 33
(2) INFORMATION FOR SEQ ID NO: 142
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 142
   GCCYCCWARR GGGGCGCCGA CGAGCGGWAT RTA 33
(2) INFORMATION FOR SEQ ID NO: 143
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 143
   AACCCGGACR CCRTGYGCCA RGGCCCTGGC AGC 33
(2) INFORMATION FOR SEQ ID NO: 144
   (i) SEQUENCE CHARACTERISTICS:
      (A) LENGTH: 33 nucleotides
      (B) TYPE; nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 144
   RTTCCCTGTT GCATAGTTCA CGCCGTCYTC CAG 33
(2) INFORMATION FOR SEQ ID NO: 145
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 145
   CARRAGGAAG AKAGAGAAAG AGCAACCRGG MAR 33
(2) INFORMATION FOR SEQ ID NO: 146
   (i) SEQUENCE CHARACTERISTICS:
      (A) LENGTH: 20 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 146
   AGGCATAGGA CCCGTGTCTT 20
(2) INFORMATION FOR SEQ ID NO: 147
   (i) SEQUENCE CHARACTERISTICS:
      (A) LENGTH: 20 nucleotides
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO: 147
   CTTCTTTGGA GAAAGTGGTG 20